(19) Europäisches Patentamt
European Patent Office
Office européen des brevets

(11) EP 4 076 560 B1

(12) **EUROPEAN PATENT SPECIFICATION**

(45) Date of publication and mention of the grant of the patent:
**01.04.2026 Bulletin 2026/14**

(21) Application number: **20828354.9**

(22) Date of filing: **17.12.2020**

(51) International Patent Classification (IPC):
***A61L 29/08** (2006.01)* ***A61L 29/14** (2006.01)*
***A61L 31/10** (2006.01)* ***A61L 31/14** (2006.01)*
***C09D 183/04** (2006.01)*

(52) Cooperative Patent Classification (CPC):
(C-Sets available)
**A61L 29/085; A61L 29/14; A61L 31/10; A61L 31/14; C09D 183/04; C09D 183/06; C09D 183/08;** A61L 2400/10; A61L 2420/02; C08G 77/16; C08G 77/18; C08G 77/24; C08G 77/26
(Cont.)

(86) International application number:
**PCT/IB2020/062119**

(87) International publication number:
**WO 2021/124206 (24.06.2021 Gazette 2021/25)**

(54) **SURFACE MODIFYING COATING FOR MEDICAL DEVICES**
OBERFLÄCHENMODIFIZIERENDE BESCHICHTUNG FÜR MEDIZINPRODUKTE
REVÊTEMENT DE MODIFICATION DE SURFACE POUR DISPOSITIFS MÉDICAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2019 US 201962952124 P**
**19.11.2020 US 202063115641 P**

(43) Date of publication of application:
**26.10.2022 Bulletin 2022/43**

(73) Proprietor: **Hospira, Inc.**
**Lake Forest, Illinois 60045 (US)**

(72) Inventors:
- **LEE, Yann-Per**
  **Lake Forest, Illinois 60045 (US)**
- **RUSH, Benjamin L.**
  **Lake Forest, Illinois 60045 (US)**
- **ZHOU, Ming**
  **Lake Forest, Illinois 60045 (US)**

(74) Representative: **Pfizer**
**European Patent Department**
**23-25 avenue du Docteur Lannelongue**
**75668 Paris Cedex 14 (FR)**

(56) References cited:
**EP-A1- 3 564 330**
**WO-A1-2014/052792**
**WO-A2-2004/064901**
**WO-A2-2007/115156**
**US-A1- 2003 216 779**
**US-A1- 2005 240 223**
**US-B2- 8 075 995**

- **N.N.: "NuSil SILICONE TECHNOLOGY", 11 June 2014 (2014-06-11), pages 1 - 13, XP055782599, Retrieved from the Internet <URL:https://www.siliconeelastomers.co.uk/pdf/Restricted_Healthcare_Materials_Selection_Guide.pdf> [retrieved on 20210305]**
- **DATABASE WPI Week 200947, Derwent World Patents Index; AN 2009-L22924, XP002802282**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 076 560 B1

(52) Cooperative Patent Classification (CPC): (Cont.)

C-Sets
**A61L 29/085, C08L 83/04;**
**A61L 31/10, C08L 83/04;**
**C09D 183/04, C08L 83/00;**
**C09D 183/06, C08L 83/00;**
**C09D 183/08, C08L 83/00**

## Description

BACKGROUND OF THE INVENTION

Field of Invention

The present invention is directed to a syringe and components thereof which are coated with a surface lubricant composition. One or more lubricious coating formulations, methods of making and applying such formulations and methods of using the same to reduce maximum static friction force and stiction between two slidable surfaces are described herein.

Description of Related Art

Today, using a combination product, such as a prefilled syringe or an on-body injector, for parenteral administration of pharmaceutical product is one of the most popular methods for unit dose medication as the pharmaceutical industry seeks new and more efficient drug delivery methods. In some cases, pharmaceutical companies can minimize drug waste and increase product shelf life or life span, while patients are able to self-administer injectable drugs at their home instead of the hospital. Compared with traditional vial and ampoules, a prefilled syringe or an on-body injector can offer many advantages such as high dosage accuracy, easy and fast handling, reduced risk of product contamination, and improved efficiency of workflow in the hospital. For example, anesthesia workflows require use of multiple on demand medications that are titrated in small volume increments. Hospitals currently source ready-to-use anesthesia or other medications from external high cost third party compounders. A prefilled syringe can reduce these extra steps and provide a cost-effective sterile injectable solution for the patients.

Even when prefilled syringes or on-body injectors are used in a hospital or clinic setting, the hospital or clinic must often take extra precautions or steps to ensure more accurate dosing. Many times, the hospital or clinic must send the empty or prefilled syringe to a special laboratory or compounding company to draw up the medication from a drug vial or pre-push down the plunger of a prefilled syringe or on-body injector to overcome the initial static friction forces and provide the precise dose desired. These extra steps add transportation and compounding costs, as well as result in shortened shelf life once the original prefilled syringe or injector is removed from its packaging.

Multiple factors can affect system performance of a prefilled syringe or an on-body injector. Among them, relatively high break loose force is a well-known problem, especially with respect to achieving accurate dosing. Break loose force and extrusion force are frictional forces between contact surfaces of two objects, for example, a plunger and a barrel of a syringe. Break loose force is a static friction force that is necessary to overcome maximum static friction force and initiate the sliding motion; while extrusion force is a kinetic friction force to continuously slide two objects relative to each other at a certain speed. Excessive break loose force can lead to difficult or incomplete dosing, reduced dosing accuracy, or even failure of the device through breakage, leakage of fluid, or jamming. Another problem is "stiction", which comes from a large discrepancy between break loose force and extrusion force. When stiction happens, the plunger can have a sudden and rapid movement, which can lead to an unpredictable or inaccurate dosing for a partial-dosing prefilled syringe. This can make the syringe less desirable for a patient who greatly relies on accurate and consistent low dosage of medication, for example a Diabetes I patient or a pediatric patient who might use a smaller dosage because of their lower body mass index.

Factors that contribute to break loose force include but are not limited to compressive force applied on the surface, substrate material and surface property.

A lubricant is sometimes used between two engaged surfaces to reduce friction force. However, although surfaces are lubricated, and friction force is reduced originally, the low break loose force may not be able to be maintained for a prolonged time and gradually increases back. The origin of a time-dependent break loose force is due to gradual lubricant migration and squeeze-out. When two objects are compressed against each other, lubricant tends to migrate and squeeze out from the contact surfaces. As more lubricant migrates out, less lubricant remains on the contact surface area, and higher break loose force is required. Then, as movement gets onto a better lubricated area, friction force suddenly drops. Stiction can happen; in other words, high differences between break loose and extrusion forces. Some in the art have recognized that lubricant migration and squeeze-out ("depletion") are dependent of compression force, size of contact area, viscosity and molecular structure of lubricant, sterilization and storage condition. Various measures have been tried in many of these areas with varying degrees of success, but no clearly superior solutions have yet to be proposed.

Various methods have been reported to improve surface lubricity and reduce break loose force. Attempts have been made to reduce migration of lubricant from contact surfaces to reduce break loose force and extrusion force using gas plasma as an energy source to treat a lubricant applied to one or more of the surfaces. Information about such gas plasma treatment of the lubricant substrate surface can be found in the following U.S. Pat. No.7,553,529; No. 7,431,989; No.

8,124,207; No. 9,133,412; No. 9,315,757. However, each of these patents suffers from a disadvantage of using a gas plasma energy source as an additional secondary operation to treat a lubricant or contact surface one or multiple times. The additional operation step or steps of plasma treatment can increase manufacture cost and complexity. The gas plasma treated surface has very limited shelf life before a lubricant has to be applied, which further complicates the manufacturing process. In addition, a specific range of appropriate lubricant viscosity to be applied was not discussed or suggested.

U.S. Pat. No. 7,332,227 discloses a three-siloxane polymer lubricant including two non-curable polymers and one curable polymer. However, one non-curable siloxane polymer has a very low viscosity, less than 50 centistokes, may lead to a significant lubricant migration and generation of lubricant particulate, which can interact with drug and compromise medication efficacy when used in a prefilled syringe. In addition, it requires UV light radiation to initiate curing reaction, which can increase operation complexity and cost.

U.S. Pat. No.8,075,995; No. 9,234,118; No. 8,603,638; No. 8,816,022; E.P. Pat. No. 2,061,529 disclose a coating, which is composed of two different curable organopolysiloxanes, one had two alkenyl groups and the other one had at least two polar groups. The curing reaction was free radical polymerization requiring an additional curing system and energy source to initiate the reaction.

U.S. Pat. No. 4,720,521 discloses a film-forming siloxane lubricant composition comprising a reactive component having three chemically crosslinked siloxane polymers, and a non-reactive component. The crosslinking reaction needed to be initiated by additional heat.

U.S. Pat. No. 9,434,857 discloses a lubricious, silicone coating composition, comprising a cross-linkable silicone polymer, a non-crosslinkable silicone polymer, a silicone cross-linking agent, and a platinum catalyst. The platinum is required as a curing catalyst but inhibits cross-linking at ambient temperatures. The reactive functionality of the curable silicone is two. Therefore, reactive silicone reacts with each other in two dimensions and a 3-dimension crosslinked network after curing reaction is not formed.

U.S. Pat. No. 5,908,686 discloses a mixture of crosslinked polysiloxanes for a lubricant coating having a thickness of 0.1 to 50 $\mu$m for a sanitary rubber article. The curing reaction is free radical polymerization and requires an additional catalyst to initiate the reaction. The number of functional groups on the curable silicone molecule that participates in the curing reaction is not reported.

U.S. Pat. No. 6,890,345 discloses four types of silicone coating: (i) one curable silicone, Dow Corning MDX4-4159; (ii) one high molecular weight non-curable polydialkylsiloxane having a molecular weight to provide a viscosity of the coating mixture of at least 10,000 cP and a curable silicone; (iii) one non-curable polydialkylsiloxanes and one high molecular weight curable silicone, Nusil MED1-4162; and (iv) two curable silicones, MDX4-4159, and high molecular weight curable silicone MED1-4162. The needle surface that the silicone-containing coating is applied to is required to be pre-treated by acid. Standalone silicone (i) is curable but does not form an interpenetrating network. Silicone mixtures (ii) and (iii) have one silicone with a high molecular weight that cannot chemically bond with a second silicone. This causes the high molecular weight silicone to tend to self-aggregate and form a non-homogenous mixture and loosely connected interpenetrating network. Silicone mixture (iv) has two self-curable silicones, and one of them has a high molecular weight. It is believed that, because the second silicone is self-curable, it would tend to aggregate or cure by or with itself and not form a very homogeneous interpenetrating network, either. Thus, this reference fails to disclose or suggest a combination of two silicones wherein the second silicone has a high molecular weight and can only copolymerize with the first silicone (and not itself) in order to provide a highly homogeneous interpenetrating network.

Objectives of the Invention

Thus, a primary objective of the present invention is the provision of syringe comprising a lubricant coating formulation that is stable and adheres well to an entire substrate surface of interest to reduce maximum static friction force and stiction for a prolonged period. The lubricant coating formulation is applied to at least one of the barrel interior surface and the outwardly directed surface of the plunger and may have several properties and advantages which are described here below.

The lubricant formulation applied in the syringe of the present invention does not require a catalyst, crosslink agent, or additional energy source to initiate the curing reaction.

The lubricant formulation applied in the syringe of the present invention forms at least a semi-impenetrable network having a three-dimensional cross-linked silicone network.

The lubricant formulation applied in the syringe of the present invention comprises a low viscosity silicone composition easily cross-linked with itself and with a high viscosity silicone composition.

The lubricant formulation applied in the syringe of the present invention, when applied to at least one of the plunger or the barrel of the syringe, reduces the break loose force required to initially move the plunger within the barrel.

The lubricant formulation applied in the syringe of the present invention, when applied to at least one of the plunger or barrel of a syringe, reduces the extrusion force or secondary break loose force required to move or reinitiate movement of the plunger within the barrel after the plunger has been moved initially and then parked for a predetermine

parking time.

The lubricant formulation applied in the syringe of the present invention reduces stiction between two adjacent sliding surfaces.

The lubricant formulation applied in the syringe of the present invention has an effectiveness that is substantially independent of the non-zero thickness of the coating layer, such that excess formulation volume or layers are not required.

The lubricant formulation applied in the syringe of the present invention is also easy and cost-effective to make.

The lubricant formulation applied in the syringe of the present invention is stable in storage for up to 180 days at room temperature and has a shelf life when applied to a syringe for up to 24 months.

The lubricant formulation applied in the syringe of the present invention is cross-linkable in the presence of moisture and at ambient or room temperature.

The lubricant formulation applied in the syringe of the present invention has a constituent that is an organopolysiloxane polymer that has a predetermined and pre-selected functional group and that can react with itself and with a second organopolysiloxane polymer that has a predetermined and pre-selected functional group.

The lubricant formulation applied in the syringe of the present invention has a second constituent that is an organopolysiloxane polymer that has a predetermined and pre-selected functional group and that can only react with a first organopolysiloxane polymer that has a predetermined and pre-selected functional group.

The lubricant formulation applied in the syringe of the present invention minimizes the differences between break loose and extrusion forces.

The ratio between break loose and extrusion forces approaches 3:1 to 1:1.

Another objective of the invention is thus the provision of an empty or prefilled syringe that has low break loose forces.

Another objective of the invention is the provision of an empty or prefilled syringe that has low extrusion forces.

Another objective of the invention is the provision of an empty or prefilled syringe that provides a consistently low break loose and extrusion force, which allows a user to improve the accuracy of single complete volume doses and multiple partial volumes doses.

Another objective of the invention is the provision of an empty or prefilled syringe that is easy for a user to use for single complete volume doses or multiple partial volume doses.

Another objective of the invention is the provision of a pre-lubricated empty or prefilled syringe that helps hospitals and clinics prepare more precise doses and thereby avoids the need to have third parties prepare or draw up more precise doses at extra costs.

Another objective of the invention is the provision of a lubricated prefilled syringe, that is capable of withstanding terminal sterilization with the drug or medication contained therein, including but not limited to steam heat autoclave sterilization at temperatures as high as 121 degrees C for 30 minutes.

These and other objectives will be apparent to those skilled in the art from the drawings, description and the claims that follow.

## SUMMARY OF THE INVENTION

The present invention is directed to a syringe comprising a lubricious coating as defined in the claims.

In some embodiments, the surface lubricant composition applied in the syringe of the present invention comprises: 1) a hydrolyzable first organopolysiloxane capable of crosslinking reaction upon exposure to moisture at ambient temperature and having a viscosity of less than or equal to 1,000 centistokes; and 2) a second organopolysiloxane that is copolymerizable with the first organopolysiloxane and has viscosity of greater than or equal to 5,000 centistokes.

In some embodiments, the surface lubricant composition applied in the syringe of the present invention comprises: 1) a hydrolyzable first organopolysiloxane capable of crosslinking reaction upon exposure to moisture at ambient temperature and having a viscosity of less than or equal to 1,000 centistokes; and 2) a second organopolysiloxane that is copolymerizable with the first organopolysiloxane and has viscosity of greater than 10,000 centistokes.

In some embodiments the surface lubricant composition applied in the syringe of the present invention includes an organic solvent in which the first organopolysiloxane and the second organopolysiloxane are mixed to form a homogeneous liquid lubricious solution. The organic solvent can include an aromatic solvent, an aliphatic solvent or both to dissolve the organopolysiloxanes. For example, the solvent can include xylene and heptane.

In some embodiments, the second organopolysiloxane is nonhydrolyzable, is not crosslinkable with itself and is only copolymerizable with said first organopolysiloxane.

The moisture can be water added in various forms to initiate crosslinking reaction between the hydrolyzable first siloxane and second copolymerizable organopolysiloxane. In one embodiment, the moisture is drawn from air exposed to the composition, the air having a relative humidity of 30% to 80% to effectively cure the composition.

In some embodiments, the hydrolyzable first organopolysiloxane comprises at least three hydrolyzable functional groups. Those groups can be independently selected from a set of hydrolyzable functional groups consisting of

acetoxy, enoxy, oxime, alkoxy, methoxy and amine. In one embodiment, at least three hydrolyzable functional groups are methoxy groups. In some embodiments, the hydrolyzable first organopolysiloxane has a viscosity of about 50 to 500 centistokes. In other embodiments, the hydrolyzable first organopolysiloxane has a viscosity of about 150 centistokes.

In some embodiments, the copolymerizable second organopolysiloxane is selected from a silanol terminated group of consisting of polydimethyl siloxane, polyalkylmethylsiloxane, polydiethylsiloxane, polyfluoropropylmethylsiloxane, polyoctylmethylsiloxane, polytetradecylmethylsiloxane, polyoctadecylmethylsiloxane, polyalkylmethyldimethylsiloxane, and polyhexadecymethylsiloxane-dimethylsiloxane. In one embodiment, the copolymerizable second organopolysiloxane is silanol terminated polydimethyl siloxane. In some embodiments, the second organopolysiloxane has a viscosity of about 30,000 centistokes. This is particularly useful when the viscosity of the first organopolysiloxane is about 150 centistokes.

In some embodiments, the lubricant composition is free of catalysts, crosslink agents, and additional energy sources to support the crosslinking reaction between the hydrolyzable first organopolysiloxane and the second organopolysiloxane. In another embodiment, the lubricant composition includes an amino functional group in the hydrolyzable first organopolysiloxane to promote adhesion to a substrate surface and accelerate the crosslinking reaction.

In one embodiment, the surface lubricant composition includes: a hydrolyzable first organopolysiloxane capable of crosslinking reaction upon exposure to moisture and having a viscosity of about 150 cSt; a second organopolysiloxane that is copolymerizable with the first organopolysiloxane and has viscosity of about 30,000 cSt; a solvent comprising of xylene and heptane to mix the organosiloxanes to form a homogeneous liquid solution; and water to trigger crosslinking between first organopolysiloxanes and copolymerization between first and second organopolysiloxanes; and the composition is free of catalysts, crosslink agents, and additional energy sources to support crosslinking between the hydrolyzable first organopolysiloxane and the second organopolysiloxane.

In other embodiments the surface lubricant composition can consist of or consist essentially of: a hydrolyzable first organopolysiloxane capable of crosslinking reaction upon exposure to moisture and having a viscosity of about 150 cSt; a second organopolysiloxane that is copolymerizable with the first organopolysiloxane and has viscosity of about 30,000 cSt; a solvent comprising of xylene and heptane to mix the organosiloxanes to form a homogeneous liquid solution; and water to trigger crosslinking between first organopolysiloxanes and copolymerization between first and second organopolysiloxanes.

In one embodiment, which can be combined with any of the other embodiments discussed herein, the hydrolyzable first organopolysiloxane is Nusil MED10-4161 and said second organopolysiloxane is Nusil MED-4162.

The surface lubricant composition disclosed herein makes possible an injectable pharmaceutical product comprising a pharmaceutically active ingredient contained in a syringe, wherein a least one component of the syringe is coated with the surface lubricant composition to reduce break loose force, the difference between it and extrusion force, and the ratio between break loose and extrusion force.

The invention provides a syringe including: a tubular syringe barrel having an interior surface; a plunger slidingly mountable within the syringe barrel and having an outwardly directed surface for slidably engaging the interior surface of the syringe barrel; the interior surface of the syringe barrel and the plunger defining a container chamber configured to contain a medicament; and a lubricious coating applied to at least one of the syringe barrel interior surface and the outwardly directed surface of the plunger. The lubricious coating is a mixture of at least two silicones comprising a first silicone being a hydrolyzable siloxane capable of crosslinking reaction upon exposure to moisture at ambient temperature and a second silicone being an organopolysiloxane that is copolymerizable with the first silicone. Thus, when a force is applied to the plunger by a user to slidably move the plunger within the syringe barrel, a break loose force to be overcome to start movement of the plunger within the syringe barrel is less than or equal to three times an extrusion force to be overcome when the plunger is already in motion.

The lubrication of the interface between a first component having a surface in frictional engagement with a surface of a second component, may be performed by a method comprising the steps of:

a. Applying the coating as defined herein to at least a portion of at least one surface of the component(s) to form a coating upon the portion of the surface; and
b. Exposing the coating of step (a) to moisture at ambient temperature to partially cure the coating.

The reduction of break loose force and stiction of slidable surfaces in the interior of a syringe assembly may be performed by a method comprising the steps of:

a. Applying a coating as defined herein to the surface of the syringe plunger to form a coating; and
b. Exposing the coating of step (a) to moisture at ambient temperature to partially cure the coating.

The surface modifying coating as described herein is for a syringe comprising a first component having a surface in frictional engagement with a surface of a second component. In accordance with the system of the invention, the force

required to achieve breakout, or break loose force can be greatly reduced, whereby transition of surfaces from stationary contact to sliding contact occurs without a sudden stiction. When breakout or break loose is complete and the surfaces are in sliding contact, they slide smoothly under low extrusion force. Substantially less lubricant may be required, and lubricant migration and silicone particulate is reduced or eliminated. The effect achieved by the system of the present invention can be of long duration, and syringes can retain the advantages of low break loose forces throughout a parking period. When the surfaces are part of a liquid dispensing device, small highly accurate increments of liquid may be dispensed repeatedly without sudden stiction. Thus, a syringe treated with the lubricant of the invention can be used to administer a medication to a patient without the risk of stiction and over or under dosing administration. Therefore, patient safety and treatment efficacy can be greatly enhanced.

Thus, in some embodiments, the syringe of the present invention may be used in a method for delivering a pharmaceutical product comprising:

providing the syringe comprising:

a tubular syringe barrel having an interior surface;
a plunger slidingly mountable within the syringe barrel and having an outwardly directed surface for slidably engaging the interior surface of the syringe barrel;
the interior surface of the syringe barrel and the plunger defining a chamber containing a medicament; and
a lubricious coating applied to at least one of the syringe barrel interior surface and the outwardly directed surface of the plunger;
wherein the lubricious coating is a mixture of at least two silicones comprising a first silicone being a hydrolyzable siloxane capable of crosslinking reaction upon exposure to moisture at ambient temperature and a second silicone being an organopolysiloxane that is copolymerizable with the first silicone;

applying a distally directed force on the plunger in order to deliver the pharmaceutical product from the chamber of the injection system, whereby the distally directed force necessary to break loose and initially move the plunger from an at rest position in the syringe barrel is less than twice a distally directed extrusion force needed to continue movement of the plunger within the syringe barrel once the plunger is moving.

BRIEF DESCRIPTION OF THE DRAWINGS

FIG. 1 is an exploded perspective view of a syringe according to the present invention;

FIG. 2 is a longitudinal cross section of the syringe of FIG. 1, assembled and filled with a medication in the syringe barrel;

FIG. 2A is an enlarged partial cross-sectional view of the plunger and syringe barrel contact area taken from FIG. 2.

FIG. 3 is a diagram depicting the chemical structure of two example organopolysiloxane molecules that may be used in the lubricious coating of the syringe of the present invention;

FIG. 4 is a simplified pictorial representation of a prior art lubricant molecule on the left-hand side of the figure and one example of a new lubricant molecule on the right-hand side of the figure that may be used in the lubricious coating of the syringe according to the present invention;

FIG. 5 is a flowchart depicting a process of making the lubricant formulation used in the syringe according to the present invention;

FIG. 6 is a flowchart depicting an exemplary process for coating plungers for use in syringes according to the present invention;

FIG. 7 is a flowchart depicting an exemplary process for coating and preparing for use a syringe barrel;

FIG. 8 is a graph illustrating plunger movement in millimeters versus force in Newtons applied to the plunger for a representative case and shows various states and force profile or characteristics with respect to a syringe or other moving plunger system;

FIG. 8A is a graph, which assumes the same general force profile of FIG. 8, but illustrates the improved results found according to one embodiment of the invention from Table 1;

FIG. 9 is a bar chart showing break loose force comparison testing between the invention and various on-market plunger/syringe combinations from Table 1;

FIG. 10 is a bar chart showing the calculated break loose force minus extrusion force values for the invention and various on-market plunger/syringe combinations from Table 1;

FIG. 11 is a bar chart showing the calculated ratio of break loose force to extrusion force values for the invention and various on-market plunger/syringe combinations from Table 1;

FIG. 12 is a bar chart showing break loose force comparison testing between several embodiments of the invention and various on-market plunger/syringe combinations from Tables 1 and 2; and

FIG. 13 is a bar chart showing break loose force comparison testing between the several other embodiments of the invention and various on-market plunger/syringe combinations from Tables 1 and 2.

## DETAILED DESCRIPTION OF THE INVENTION

In the description that follows it will be helpful to define certain terms for the reader to better understand the disclosure.

**Distal and proximal ends-** A syringe, carpule, on-body injection, and other articles for delivering medication normally include two generally opposite ends: a distal end and a proximal end. The distal end is usually connected with a needle, a needle hub, or a luer lock to deliver or withdraw fluid through it because the distal end is the end that is placed nearest to the site of medication delivery or the patient's skin. The proximal end is generally opposite of the distal end and has the larger open end that allows a plunger forced by a plunger rod or shaft to slide inside the barrel. The proximal end may optionally include a finger flange or grip.

**Ambient temperature** means about 20 to 25 degrees Celsius, with an average of approximately 23 °C.

**High temperature sterilization,** by steam or other means, refers to a sterilization cycle that is operated at a temperature higher than 100°C. The most common example of high temperature sterilization is steam sterilization, which is typically done at a temperature from 110 °C to 125 °C.

**Catalyst** means a substance that increases the rate of a chemical reaction without itself undergoing any permanent chemical change.

**Cross-linking agent or curing agent** means a chemical product that form chemical bonds between two molecules.

**cP** is an abbreviation for centipoise, a centimeter-gram-second unit of measurement for the dynamic or absolute viscosity of a fluid. A centipoise is 1/100 or 0.01 of a poise. A poise is 1 gram per centimeter-second. Thus, one centipoise is equal to 0.001 newton second per square meter.

**cSt** is an abbreviation for centistokes, a centimeter-gram-second unit of measurement for the kinematic viscosity of a fluid. 1 centistoke (cSt) is equal to 1 $mm^2/s$ or $10^{-6}$ $m^2/s$. The kinematic viscosity is the internal or inherent resistance of a fluid to flow with no external force applied, or in other words simply under the weight of gravity. A centistoke is 1/100 or 0.01 of a stoke. A stoke is equal to the viscosity of a fluid in poises divided by the density of the fluid in grams per cubic centimeter. Unless otherwise noted in this disclosure, kinematic viscosity is the type of viscosity being discussed.

**Break loose force** is a static friction force that is necessary to overcome maximum static friction force and initiate the sliding motion;

**Extrusion force** is a kinetic friction force to continuously slide two objects relative to each other at a certain speed. It is also sometimes referred to in the art as the gliding force.

**Secondary break loose force** after parking period is the break loose force that a syringe plunger needs to overcome after an intermission period during which no movement of the plunger takes place.

**Parking period** is an intermission period in which a syringe plunger is not in motion.

**Syringe** in this description is used broadly enough to encompass a carpule, reservoir, container or cartridge. A cartridge is a small container for powder, liquid, or gas, made for ready insertion into some device.

**Functional groups** are specific substituents or moieties within molecules that are responsible for the characteristic chemical reactions of those molecules. The same functional group will undergo the same or similar chemical reaction regardless of the size of the molecule it is a part of.

**Stiction** means the tendency of two surfaces in stationary contact to develop a degree of adherence to each other.

**Overdose** is the ingestion or application of a drug or other substance in quantities greater than what is recommended or prescribed.

**Underdose** is the ingestion or application of a drug or other substance in quantities less than what is recommended or prescribed.

As used herein, the term "cure" as used in connection with a composition, e.g., a "cured composition" or a "cured coating" shall mean that at least a portion of the crosslinkable components which form the composition are at least partially crosslinked. As used herein, the term "**curable**", as used in connection with a component of the composition, means that the component has functional groups capable of being crosslinked.

As used herein, the term "**condensation polymerization**" is a form of step-growth polymerization. Small molecules react with each other to form larger structural units while releasing smaller molecules as a byproduct, such as water or methanol.

As used herein, the term "**free radical polymerization**" is a method of polymerization by which a polymer forms by the successive addition of free-radical building blocks. Free radicals can be formed by several different mechanisms, usually involving separate initiator molecules. Following its generation, the initiating free radical adds (nonradical) monomer units, thereby growing the polymer chain.

As used herein, the term "**Interpenetrating Network (IPN)**" refers to a type of polymer system in which two chemically distinct networks coexist, ideally having a structure that is homogeneous down to the segmental level. The two components are present as co-continuous, interlocking networks. IPNs are referred to full IPNs and Semi IPNs, depending on whether both respective components (Full IPNs) or only one (Semi IPN) is crosslinked.

The term "**alkyl**", as used herein, means a straight or branched chain monovalent hydrocarbon group of formula $-C_nH_{(2n+1)}$. Non-limiting examples include methyl, ethyl, propyl, butyl, 2-methyl-propyl, 1,1-dimethylethyl, pentyl and hexyl.

The term "**cycloalkyl**", as used herein, means a cyclic, monovalent hydrocarbon group of formula $-C_nH_{(2n-1)}$ containing at least three carbon atoms. Non-limiting examples include cyclopropyl, cyclobutyl, cyclopentyl and cyclohexyl.

In some instances, the number of carbon atoms in a hydrocarbon group (e.g. alkyl, alkenyl, alkynyl, cycloalkyl or alkylene) is indicated by the prefix "$C_x$-$C_y$" or "$C_{x-y}$", wherein x is the minimum and y is the maximum number of carbon atoms in the group. Thus, for example, "($C_1$-$C_6$)alkyl" or "$C_{1-6}$ alkyl" refers to an alkyl substituent containing from 1 to 6 carbon atoms.

The term "**alkoxy**", as used herein, means an alkyl substituent attached through an oxygen atom. Non-limiting examples include methoxy, ethoxy, propoxy, butoxy, pentoxy, and hexyloxy.

The term "**acetoxy**", as used herein, which is represented by the formula $-O-C(=O)-CH_3$, also known an acetyloxy group.

The term "**aryl**", as used herein, means phenyl or naphthalenyl.

The term "**aralkyl**", as used herein, refers to an alkyl group substituted by one to five aryl groups.

The term "**formyl**", as used herein, means a -C(O)H group.

The term "**haloalkyl**", as used herein, refers to an alkyl group that is substituted with at least one halogen substituent. Where more than one hydrogen atom is replaced with a halogen atom, the halogens may be identical or different. Non-limiting examples include fluoromethyl, difluoromethyl, trifluoromethyl and 2,2,2-trifluoroethyl.

The term "**haloaryl**", as used herein, refers to an aryl group that is substituted with at least one halogen substituent. Where more than one hydrogen atom is replaced with a halogen atom, the halogens may be identical or different.

The term "**amino**", as used herein, refers to $-NH_2$.

The term "**amine**", as used herein, refers to a -NRR' group, where R and R' are independently H, ($C_1$-$C_6$)alkyl, aryl, or haloalkyl.

The term "**enoxy**", as used herein, refers to a $-O-C(CH_3)(=CH_2)$ group.

The term "**oxime**", as used herein, refers to a subgroup of imines represented as a -ON=C(CH3)(C2H5), wherein R is hydrogen, ($C_1$-$C_6$)alkyl, aryl, aryl ($C_1$-$C_6$)alkyl, or haloaryl.

When a substituent is defined as a combination of two groups (e.g. **alkoxyalkyl**) the moiety concerned is always attached through the second of the two groups named (in this case alkyl). Thus, for example, ethoxymethyl corresponds to $CH_2CH_3-O-CH_2-$.

As illustrated in FIG. 1, the medical device is a syringe assembly 10 comprising a first component which is a syringe barrel 12, and a second component which is a plunger 14. The syringe assembly 10 in some embodiments can include additional components and features including but not limited to a plunger rod 16 that has a proximal end 18 defining a thumb push button 20 and a distal end 22 that is couplable to the plunger 14. The coupling is provided by mating coupling means 24 and 26 on the plunger rod 16 and plunger 14 respectively. In the embodiment shown in FIGS. 1 and 2, the mating coupling means 26 on the plunger 14 is a threaded or slotted cavity 15 in the proximal end 17 of the plunger 14 and the mating coupling means 24 on the distal end 22 of the plunger rod 16 is a threaded or lugged projection 19. However, one of skill in the art will understand that the cavity could be in the distal end of the plunger rod 16 and the projection could be on the

proximal end of the plunger 14. In addition or alternatively, adhesive, welding, an interconnecting insert, or other mechanical interlocking features could be used to couple the plunger 14 and plunger rod 16 together so that they move axially substantially as a single integrated unit once coupled.

As best seen in FIG. 2, the syringe barrel 12 is a hollow tubular structure. In one embodiment the syringe barrel 12 is cylindrical, elongated, and has a wall 28 defining an exterior surface 30 and an interior surface 32 extending between a reduced diameter distal end 34 and an enlarged proximal end 36. The enlarged proximal end 36 includes a pair of laterally extending, opposing finger flanges 38 and 40 formed thereon, as best seen in FIG. 1. As best seen in FIGS. 2 and 2A, the interior surface 32 has a retention rib 42 protruding radially inward adjacent to the proximal end 36. The proximal end 36 has a central opening 44 therein for receiving the plunger 14 into the cavity 46 surrounded by the interior surface 32 of the syringe barrel wall 28. The cavity 46 is adapted to receive a medicament 5 and thus acts as a reservoir for the medicament 5. The cavity 46 is connected to an outlet opening 48 by a small passageway 50 in the reduced diameter distal end 34. The reduced diameter distal end 34 includes a mount 52 for an optional needle hub assembly (not shown) or the mount 52 can simply be a tapered or straight, male or female luer connection for needleless delivery. The syringe barrel in one embodiment is formed of a polypropylene material suitable for medical applications such as ExxonMobil™ PP9074MED or BORMED™ HD810MO.

As best understood in view of FIGS. 1, 2 and 2A, the plunger 14, which is preferably formed from an elastomeric material such as a thermoplastic elastomer or, more preferably for medical applications, a synthetic thermoset elastomer such as butyl rubber, ethylene propylene diene monomer (EPDM) rubber, silicone rubber, acrylonitrile butadiene (Buna-N) rubber, and polyisoprene rubber. In one embodiment, the plunger 14 is formed from a butyl rubber available under the trade designation FM457/0 Gray from Datwyler of Belgium. The plunger 14 has one or more slidable sealing surfaces or protruding annular ring portions 54A, 54B, 54C. The surface(s) seal with the interior surface 32 of the syringe barrel 12 when the plunger 14 is inserted into the barrel 12 and retain the medicament 5 in the cavity 46 until time for displacement and delivery by the syringe assembly 10. The surface(s) also slidingly move across portions of the interior surface 32 when a user or clinician applies an axially directed force F to the plunger rod 16. Only the plunger rod 16 and the plunger 14 coupled thereto move with respect to the syringe barrel 12. In the static condition before any movement the forces must be in balance according to Equation 1 below. Once in motion, the plunger 14 moves according to the dynamic equation shown in Equation 2 below.

$$\text{Equation 1:}\quad F_S - f_0 = 0;$$

where $f_0$ is the break loose force.

$$\text{Equation 2:}\quad F_D - f_1 = 0;$$

where $f_1$ is the extrusion or gliding force.

Where in Equation 1, $f_0$ is the static frictional force that must be overcome to move the plunger 14 within the syringe barrel 12 and $F_S$ is pushing force the user must apply to plunger rod 16 to initiate moving of plunger 14. And in Equation 2, $f_1$ is the dynamic frictional force that must be overcome to keep the plunger 14 moving within the syringe barrel 12 and $F_D$ is the pushing force the user must apply to the plunger rod 15 to continuously move the plunger 14.

The first component or syringe barrel 12 of the syringe assembly 10 can be formed from glass, metal, ceramic, plastic, rubber or combinations thereof. In some embodiments, the first component is prepared from one or more olefinic polymers, such as polyethylene, polypropylene, poly(1-butene), poly(2-methyl-1-pentene) and/or cyclic polyolefin. For example, the polyolefin can be a homopolymer or a copolymer of an aliphatic mono-olefin, the aliphatic mono-olefin preferably having about 2 to 6 carbon atoms, such as polypropylene. In some embodiments, the polyolefin can be basically linear, but optionally may contain side chains such as are found, for instance, in conventional, low density polyethylene. In some embodiments, the polyolefin is about 50% to 99% isotactic. In other embodiments, the polyolefin is about 90% to 99% isotactic in structure. In some embodiments, syndiotactic polymers can be used. A non-limiting example of a suitable cyclic polyolefin includes an ethylene-norbornene copolymer such as TOPAS® ethylene-norbornene copolymer commercially available from Topas Advanced Polymers of Florence, KY.

The polyolefin can contain a small amount, generally from about 0.1 to 10 percent, of an additional polymer incorporated into the composition by copolymerization with the appropriate monomer. Such copolymers may be added to the composition to enhance other characteristics of the final composition, and may be, for example, polyacrylate, polyvinyl, polystyrene and the like.

In some embodiments, the first component may be constructed of a polyolefin composition which includes a radiation stabilizing additive to impart radiation stability to the container, such as mobilizing additive which contributes to the radiation stability of the container, such as for example those disclosed in U. S. Pat. Nos. 4,959,402 and 4,994,552, assigned to Becton, Dickinson and Company.

The second component or plunger 14 of the syringe assembly 10 can be formed from any material such as discussed above for the first component, but preferably is formed from an elastomeric material. Elastomers are used in many important and critical applications in medical devices and pharmaceutical packaging. As a class of materials, their unique characteristics, such as flexibility, resilience, extendibility, and sealability, have proven particularly well suited for products such as syringe plungers, syringe tips, catheters, drug vial articles, injection sites, tubing, gloves, O-rings, and hoses. Elastomer material is consisting of thermoset elastomer and thermoplastic elastomers. Primary synthetic thermoset elastomers typically are used in medical applications: butyl rubber, silicone rubber, ethylene propylene diene monomer (EPDM) rubber, acrylonitrile butadiene (Buna-N) rubber and polyisoprene rubber. Of these rubbers, butyl rubber has been the most common choice for articles due to its high cleanness and permeation resistance to moisture and oxygen for water-sensitive drugs and oxygen-sensitive drugs respectively.

Suitable butyl rubbers useful in the method of the present invention including copolymers of isobutylene (about 97-98%) and isoprene (about 2-3%). The butyl rubber can be halogenated with chlorine or bromine. Suitable butyl rubber vulcanizates can provide good abrasion resistance, excellent impermeability to gases, a high dielectric constant, excellent resistance to aging and sunlight, and superior shock-absorbing and vibration-damping qualities to articles formed therefrom.

Thermoplastic elastomers include, without limitation, styrene copolymers such as styrene-butadiene (SBR or SBS) copolymers, styrene-isoprene (SIS) block polymer or styrene-isoprene/butadiene (SIBS), in which the content of styrene in the styrene block copolymer ranges from about 10% to about 70%, and preferably from about 20% to about 50%. The rubber composition can include, without limitation, antioxidants and/or inorganic reinforcing agents to preserve the stability of the rubber composition.

In some embodiments, the second component can be a sealing member, such as a plunger, a stopper, O-ring, plunger tip or piston. Syringe plunger tips or pistons typically are made of a compressible, resilient material such as butyl rubber, because of the vulcanized rubber's ability to provide a seal between the plunger and interior housing of the syringe. Syringe plungers, like other equipment used in the care and treatment of patients, must meet high performance standards, such as the ability to provide a tight seal between the plunger and the barrel of the syringe.

The coating is applied to at least a portion of at least one surface of the component(s) in frictional engagement with an opposed surface of another component. In some embodiments, at least a portion of at least one surface of the component(s) is coated with the coating. In other embodiments, at least a portion of a surface of the first component and at least a portion of a surface of the second component are coated with the coating. In some embodiments in which the first component is prepared from a non-cyclic polyolefin, the portion of the surface of the first component is uncoated, and the portion of the surface of the second component is coated with the coating. In some embodiments in which the syringe barrel is prepared from polypropylene and sealing member is prepared from butyl rubber, the surface of the syringe barrel is uncoated and the at least one portion of the surface of the sealing member is coated with the coating. In other embodiments in which the syringe barrel is prepared from polypropylene and the sealing member is prepared from butyl rubber, the at least one portion of the surface of the sealing member can be coated with the coating. Methods for coating the surface(s) are discussed in detail below. One skilled in the art will appreciate that the sealing member could be selected from a group consisting of a syringe plunger, stopper, O-ring, plunger tip and piston.

The coating used in the present invention comprises a partially cured coating prepared from a coating composition of a mixture of at least two silicones, including: (i) a hydrolyzable organopolysiloxane that has a viscosity of less than or equal to 1,000 centistokes and is capable of crosslinking reaction upon exposure to moisture at ambient temperature; and (ii) a second organopolysiloxane that is copolymerizable with the first hydrolyzable organopolysiloxane, and has viscosity of greater than or equal to 5,000 centistokes. In another embodiment, the second organopolysiloxane has a viscosity of greater than 10,000 centistokes. The coating provides a maximum static friction force equal to or less than two times of kinetic force to reduce stiction between engaged surfaces. The coating is compatible with high-temperature sterilization method and long-term aging conditioning and does not require a catalyst, crosslink agent, or additional energy source.

The degree of crosslinking, ranges from 1% to 99% of complete crosslinking. One skilled in the art will understand that the presence and degree of crosslinking, i.e., the crosslinking density can be determined by a variety of methods, such as dynamic mechanical thermal analysis (DMTA) using a Hitachi DMS7100 DMTA analyzer conducted under nitrogen. This method determines the glass transition temperature and crosslink density of coating. The physical properties of a cured material are related to the structure of the crosslinked network.

As discussed above, the coating used in the present invention comprises a partially cured coating prepared from a composition comprising one curable organopolysiloxane having more than two reactive alkoxy functionalities on the molecule. In other words, this organopolysiloxane must contain at least three hydrolyzable functional groups as shown below.

In some embodiments, the first organopolysiloxane can be represented by the following structural formulae (I)

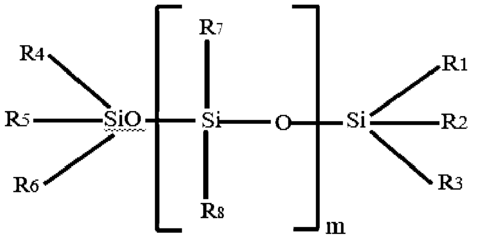

I

At least two of R1, R2, R3, R4, R5 and R6, are selected independently from the group consisting of acetoxy, enoxy, oxime, and ($C_1$-$C_6$)alkoxy. The rest of R1 - R6 can be amine groups or ($C_1$-$C_6$)alkyl groups. R7 and R8 are independently ($C_1$-$C_6$)alkyl, halo($C_1$-$C_6$)alkyl, aryl, haloaryl, ($C_3$-$C_7$)cycloalkyl, ($C_1$-$C_6$)aralkyl, fluoro, fluoro($C_1$-$C_6$)alkyl, and mixtures thereof; m is about 5 to about 1000, more preferably about 200 to about 320. Copolymers of these polymers are also contemplated.

Non-limiting examples of a suitable first organopolysiloxane of the formula (I) include MED-4159 and MED10-4161 silicone dispersions available from Avantor subsidiary Nusil Technology LLC of Carpinteria, CA, MDX4-4159 50% Medical Grade Dispersion available from Dow Corning or Dow Chemical Company of Midland, MI, DMS-D33 and DMS-XT11 available from Gelest Inc. of Morrisville, PA, and KR-401 available from Shin-Etsu Silicones of America, Inc. of Akron, OH.

The first organopolysiloxane of the composition is a low viscosity organopolysiloxane polymer which has a viscosity less than about 5,000 centistokes, more desirably less than 1,000 centistokes, and preferably about 150 to 300 centistokes.

The second non-curable organopolysiloxane of the composition of the present invention is a high viscosity organopolysiloxane polymer which has a viscosity greater than or equal to about 5,000 centistokes, more desirably greater than about 12,500 centistokes such as from about 12,500 to about 1,000,000 centistokes, more desirably from about 30,000 to about 500,000 centistokes. The high viscosity organopolysiloxane polymer desirably has a number average molecular weight of from 20,000 to about 200,000, desirably from about 60,000 to about 100,000.

In some embodiments, the second organopolysiloxane can be represented by the following structural formulae (II)

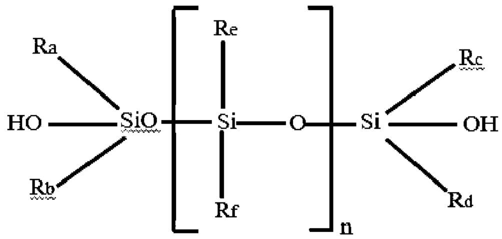

II

Where Ra, Rb, Rc, Rd, Re, and Rf are independently selected from the group consisting of ($C_1$-$C_6$)alkyl, halo($C_1$-$C_6$)alkyl, aryl, haloaryl, ($C_3$-$C_7$)cycloalkyl, ($C_1$-$C_6$)aralkyl, fluoro, fluoro($C_1$-$C_6$)alkyl, and mixtures thereof; n is about 200 to about 2000, more preferably about 600 to about 1000. Copolymers and mixtures of these polymers are also contemplated. In some embodiments, the second organopolysiloxane is copolymerizable and is selected from a silanol terminated group consisting of polydimethyl siloxane, polyalkylmethylsiloxane, polydiethylsiloxane, polyfluoropropylmethylsiloxane, polyoctylmethylsiloxane, polytetradecylmethylsiloxane, polyoctadecylmethylsiloxane, polyalkylmethyldimethylsiloxane, and polyhexadecymethylsiloxane-dimethylsiloxane.

Non-limiting examples of organopolysiloxanes useful as the high viscosity organopolysiloxane polymer having a viscosity greater than or equal to about 5,000 centistokes include DMS-S35 5,000 centistokes polydimenthylsiloxane, commercially available from Gelest Inc. of Morrisville, PA, MED-4162 30,000 centistokes polydimethylsiloxane, commercially available from Nusil Technology LLC of Carpinteria, CA, DMS-S42 18,000 centistokes available from Gelest Inc. of Morrisville, PA, and 482005 50,000 centistokes available from Millipore Sigma of St. Louis, MO, USA.

In some embodiments, the first organopolysiloxane is present in the composition at a total weight of about 1 weight% to 99 weight%, more preferably from about 5 weight % to about 75 weight%, based on the total weight of the composition.

The crosslinking reaction is a condensation polymerization. In the presence of moisture, the hydrolyzable groups react with moisture to form silicone hydroxyl groups (-Si-OH). The silicon hydroxyl groups are extremely reactive and react with other functional or hydroxyl groups present in the silicone or on the substrate by inter-molecular or intra-molecular condensation polymerization. Typical examples of curing reaction by hydrolyzable reactive groups are

Alkoxy 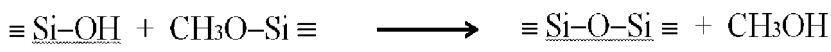

Acetoxy 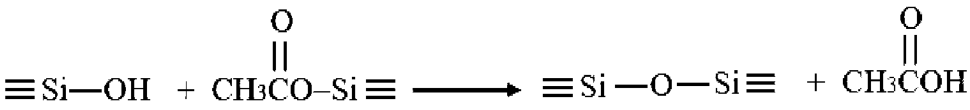

Enoxy 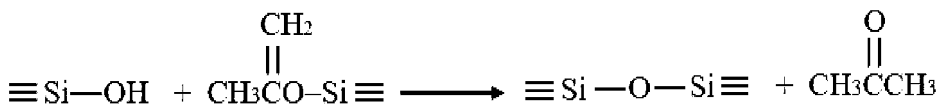

Oxime 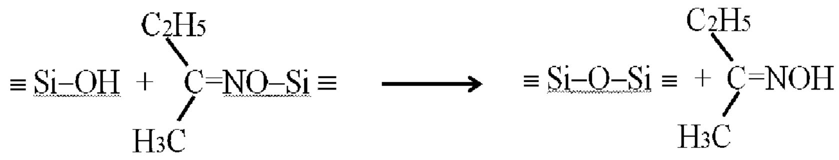

Amine 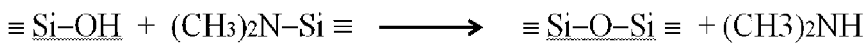

Advantageously, curing of the reactive portion can be accomplished at ambient temperature in the presence of moisture. The relative humidity level required for proper curing should be between 30% and 80%, preferably between 40% to 60%. Low humidity slows down the curing process while excess humidity inhibits the reaction resulting in a tacky surface on the treated article. With sufficient humidity the material will cure at room temperature in about 24-48 hours, preferably about 24 hours, and lubrication properties may continue to develop over a period of several days after the initial cure. To speed up the curing process for cost-effective production, one alternative option for curing is to store the coated parts at room temperature with optimum humidity for 1-2 hours, then move the parts to a heated area (50 to 60 °C) to accelerate removal of solvent and the curing process. The heating time and temperature is dependent on the heat tolerated by the part, but in any case, it is sufficient to use only enough time to remove the solvent. The curing step can optionally comprise heating the coating to a temperature of from about 50 °C to about 200 °C for about 1 hour to 6 hours to effectively crosslink the hydrolyzable siloxane and the second organopolysiloxane.

The film-forming compositions used in the present invention consist essentially of the constituents described above, but may further include other additives, such as optional crosslinking agents, adhesion promoters, coupling agents, dyes and pigments, antimicrobial agents, provided that such additives do not adversely affect the properties of the composition.

The above-described silicones are dispersed into organic solvent(s) to form the novel lubricious coating solutions or compositions used in the present invention. Both aromatic and aliphatic solvents, and combinations thereof, can be used for the silicone dispersions. Typical examples of useful aromatic solvents include, but are not limited to, xylene and toluene. Aliphatic solvents which are useful include, but are not limited to, heptane, hexane, isopropyl alcohol, Stoddard, alkane, petroleum ether, pentane, kerosene, diisopropyl ether, hexyl ether, ethyl acetate, butyl acetate, isopropyl laurate, isopropyl palmitate, isopropyl myristate, methyl ethyl ketone, methyl isobutyl ketone, lauryl alcohol, 3M™ Novec™ engineered fluids and their mixtures. The organic solvent is added at a concentration sufficient to provide effective mixing of the silicone polymer components into a homogeneous coating solution. The total solvent concentration sufficient to be effective is typically between about 80 wt.% to about 99.5 wt.% and is more typically between 85 wt.% and 99 wt.%, depending upon the coating thickness requirement. Those skilled in the art will appreciate that the coating thickness can be engineered by changing the silicone content of the coating solution.

The following procedure as described utilizes conventional mixing equipment in typical production facilities. One embodiment of the process for making the coating formulation of this invention is shown in FIG. 5. The coating composition of the present invention may be preferably prepared in the following manner. Initially, the basic components (also referred to herein as Silicone 1 and Silicone 2) of the silicone composition are added to a conventional mixing vessel, along with a suitable organic solvent such as heptane or a mixture of xylene and heptane, in step 500 of the process. Care should be taken to minimize the exposure time to air. In one embodiment, the process includes a preliminary sub-step of purging the vessel with nitrogen gas such that relative humidity is held at or below 10% during the mixing step. Neither component should be left exposed to air more than 30 minutes. In step 510 of the process, ingredients are mixed for a sufficiently effective time such as 0.5-6 hours, or more preferably 2-4 hours, or for example about 2 hours to form a fully homogeneous solution. This can be achieved by running the mixer at 7,000 to 8,000 RPM. It is preferred to maintain a closed system during stirring or mixing to minimize the impact of moisture on the silicone solution and prevent premature curing. In step

520, the resulting lubricant solution can be transferred to a suitable container for storage and later use or conveyed for immediate use.

FIG. 6 illustrates one embodiment of a process for coating one or more plungers 14 with the lubricant formulation of the invention. The process can be performed on plungers individually or in batches. In step 600, plungers 14 are loaded in a basket and the basket containing the plungers is loaded into a cleaning machine for small parts. One non-limiting example of such a cleaning machine is available from Atec Steritec, a subsidiary of Atec Pharmatechnik GmbH, located in Sörup, Germany. In step 610, the plungers 14 are washed and rinsed. Any metals are leached out and the plungers 14 are dried. In step 620, the silicone solution of the invention is added to a reservoir in a dip coating station of a siliconization machine. One non-limiting example of such a siliconization machine is available as a custom machine from Atec Steritec, as mentioned above. In step 630, the plungers 14 are delivered to a fine mesh basket and then to a dip coating station. In step, 640 the plungers 14 are dipped into the silicone lubricant solution for approximately 1-15 minutes, more preferably 2-10 minutes, or even more preferably 2-5 minutes. Advantageously the dip coating can be done at room or ambient temperature. In step 650, the plungers 14 are transferred to a tumbling and drying station in the same mesh basket. In step 660, the plungers 14 are tumbled and dried by forced, circulated, ambient temperature air for about 15-75 minutes, more preferably about 15-60 minutes, and even more preferably about 20-40 minutes, with about 30 minutes providing good results. In step 670, the plungers 14 are dumped out of the mesh basket and transferred to racks for curing at room or ambient temperature for about 6-72 hours, more preferably about 12-60 hours, and even more preferably about 24 - 48 hours. In step 680, the plungers 14 are transferred to an autoclave sterilization station on a tray or in a basket. In step 690, the plungers 14 are steam sterilized in an autoclave at about 110-125 °C or 230-257 °F for about 15-30 minutes. In step 695, the plungers 14 are stored in sealed plastic bags or bins and ready for use in assembly. In embodiments where needed, the plungers 14 can be coupled to the plunger rods 16 before or after the application of the silicone lubricant formulation of this invention. In some applications like carpules or other injectors, a plunger rod 16 may be supplied separately or not necessary at all.

One skilled in the art will appreciate that either the plungers 14 or the syringe barrels 12, or both, can be coated with the lubricant solution of the invention according to the process generally shown in FIG. 6 and described above.

Steps 700 and 710 of FIG. 7 show, an alternate process for coating one or more syringe barrels 12. As an alternative to dip coating, this process involves spraying the lubricant solution of this invention on the syringe barrel 12. A person of skill in the art will appreciate that such a spray coating process could also be used on the exterior surface of the plungers 14 instead of the dip coating process described above. To save material and cost, silicone solution spray can be focused just on the annular ring portions 54A, 54B and 54 C, which are the primary frictional contact surfaces on the plunger 14 that engage the barrel 12, or the spray can be applied over substantially the entire outer surface of the plunger 14.

Although the entire syringe barrel 12 can be coated with the lubricant formulation described herein, to save material and minimize cost only the portion of the interior surface 32 that is configured to be in contact with the plunger 14 needs to be coated with the lubricant formulation to benefit from it. The process can be done on an individual syringe barrel 12 or in batches to multiple barrels 12. In step 700, the syringe barrels 12 are provided and placed on a holding fixture in a silicone spray station. For example, the holding fixture can have lugged or threaded posts and the mount 52 on the reduced diameter distal end 34 of the syringe barrel 12 can be screwed to one of the holding fixture posts with the central opening 44 in the proximal end 36 of the barrel accessible or exposed. In step 710, the silicone solution of the invention is sprayed through the opening 44 and onto the desired areas of the internal or interior surface 32 of the syringe barrel 12.

The remaining steps 720, 730, 740, 750 and 760 of the process illustrated in FIG. 7 pertain to the assembly of the syringe assembly 10 and is substantially identical whether the plunger 14, syringe barrel 12, or both are dip-coated or spray-coated with the silicone formulation of the invention. In step 720, the plunger 14 is inserted to a desired depth into the proximal end 36 of the syringe barrel 12 through the central opening 44. In step 730, the syringe barrel 12 is filled through the outlet opening 48 in the distal end 34 to a predetermined, desired volume with a medicament 5 and then the opening 48 is sealed with a conventional removable tip cover (not shown). In step 740, the prefilled syringe assembly 10 is placed into an autoclave sterilization station. In step 750, the prefilled syringe assembly 10 is sterilized by pressurized steam heat. Steam sterilization or autoclaving is a common sterilization technique, which is a relatively simple process that exposes a device, for example, to saturated steam at temperatures of 110 °C to 125 °C for a minimum of twenty minutes at a pressure of about 165 kPa. The process is usually carried out in a pressure vessel designed to withstand the elevated temperature and pressure. Autoclaving kills microorganisms by destroying metabolic and structural components essential to their replication. Autoclaving is the method of choice for sterilization of combination product, which contains a drug molecule that may be sensitive to high energy irradiation dosage such as gamma irradiation or E-beam. In step 760, the prefilled, sterilized syringe assembly is ready for secondary packaging and use. As non-limiting examples, the medicant 5 can include rocuronium bromide, ephedrine, and succinylcholine chloride. The medicament 5 also can be selected from a group consisting of rocuronium bromide, ephedrine, and succinylcholine chloride.

The surfaces of the syringe of the present invention which have a sliding relationship with each other are treated with the coating which is cured. The coating may be applied to any surface which slides in contact with another surface.

Application of a film of coating to the sliding surfaces may be accomplished by any suitable method, as, for example, dipping, brushing, spraying and the like. The solvent is subsequently removed by evaporation. The lubricant film may be of any convenient thickness and, in practice, the thickness will be determined by such factors as the concentration of silicone composition, the solvent selected, and the viscosity of the lubricant. For reasons of economy, the film preferably is applied as thinly as practical with complete surface coverage (i.e., a non-zero thickness film), since no significant advantage is gained by thicker films. Ideally the coating thickness should be in the range of 0.1 to 40 $\mu$m, more preferably 1.0 to 20 $\mu$m, and most preferably 1 to 6 $\mu$m. Thus, in some embodiments, the lubrication of the interface between a first component having a surface in frictional engagement with a surface of a second component, is performed by a method comprising the steps of: (a) applying a coating formulated as described herein to at least a portion of at least one surface of the component(s) to form a coating upon the portion of the surface; and (b) crosslinking the coating of step (a) to partially cure the coating in the presence of moisture at ambient temperature.

In other embodiments, the reduction of break loose force of a surface adapted for slidable engagement with another surface is performed by a method comprising: (a) applying a coating formulated as described herein to at least a portion of at least one surface to form a coating upon the portion of the surface; and (b) crosslinking the coating of step (a) to partially cure of the coating.

In other embodiments, the reduction of stiction of a surface adapted for slidable engagement with another surface is performed by a method comprising: (a) applying a coating formulated as described herein according to the invention to at least a portion of at least one surface to form a coating upon the portion of the surface; and (b) crosslinking the coating of step (a) to partially cure of the coating.

In other embodiments, the reduction of break loose force and stiction of a surface adapted for slidable engagement with another surface is performed by a method comprising: (a) applying a coating formulated as described herein to at least a portion of at least one surface to form a coating upon the portion of the surface; and (b) crosslinking the coating of step (a) to partially cure of the coating.

In other embodiments, the reduction of break loose force and stiction of a surface adapted for slidable surfaces in the interior of a syringe assembly is performed by a method comprising: (a) applying a coating formulated as described herein to at least a portion of at least one surface to form a coating upon the portion of the surface; and (b) crosslinking the coating of step (a) to partially cure of the coating.

Break loose forces may be conveniently measured on a universal mechanical tester or on a testing machine of the type having a constant rate of cross-head movement, as, for example an Instron model 5564 or 5565, as described in detail below.

The coating used in the present invention may be applied onto a pretreated surface to enhance coating anchorage on the surface. Non-limiting example of surface pre-treatment is atmospheric plasma, vacuum plasma, Corona treatment, ionizing radiation such as gamma radiation produced by, for example, cobalt-60 and cesium-137 sources, electron beam radiation.

The coating used in the syringe of the present invention may include optional additives such as a fluorochemical compound to improve surface compatibility with drug solution. The fluorochemical compound may comprise a perfluoropolyether (PFPE). Representative examples of commercially available PFPE include Fomblin M®, Fomblin Y®, and Fomblin Z® families of lubricants from Solvway Solexis; Krytox® from Dow Chemical; and Demnum® from Daikin Industries, Limited. In various embodiments, the lubricant may comprise a functionalized PFPE. Representative examples of commercially available functionalized PFPE include Fomblin ZDOL®, Fomblin ZDOL TXS®, Fomblin ZDIAC®, Fluorolink A10®, Fluorolink C®, Fluorolink D®, Fluorolink E®, Fluorolink E10® from Solvay Solexis.

The coating system used in the syringe of the present invention may include an additional coating, such as parylene coating, silicon dioxide coating, aluminum trioxide coating to provide a surface barrier to oxygen or/and moisture for better drug stability, for example, biologics.

The coating system used in the syringe of the present invention may include an antimicrobial additive to provide surface barrier to microorganism. Antimicrobial additive may comprise chlorhexidine diacetate, chlorhexidine glycol, or silver and combinations thereof.

The present invention is more particularly described in the following examples, which are intended to be illustrative only, as numerous modifications and variations therein will be apparent to those skilled in the art.

## EXAMPLES

### Example 1 (EX. 1) - Control Group I (Conventional Low Viscosity Silicone Lubricant)

All of plungers used in Examples 1 to 18 are made from butyl rubber with a material hardness of around 50 Shore A. Thus, the rubber materials were not a critical factor in lubricant performance for these examples. In EX. 1, components of a 5 ml syringe, more particularly the plungers, made from Helvoet 5212 bromo butyl rubber, were coated with a conventional low-viscosity, 1000 centistokes, polydimethylsiloxane through dip coating, then exposed to autoclave

sterilization, and evaluated for break loose force, extrusion force and syringe stiction. The conventional silicone lubricant is commercially available from Dow Chemical Company under the trade designation DC360 Medical Fluid, 1,000 cSt. The plunger rubber material was purchased from Datwyler of Schattdorf, Switzerland.

The syringe barrels are made of a polypropylene blend, which is conventional and not particularly important for the invention. By way of example and not limitation, the syringe barrel can also be made of glass or other suitable material without detracting from the invention. Each syringe barrel was lubricated with the same conventional polydimethylsiloxane having a viscosity of 1,000 centistokes as mentioned above.

Each syringe was assembled and filled with 5 ml. of deionized water to simulate an aqueous drug solution and autoclaved at 121 °C for 30 minutes.

The break loose force (in Newtons) of each sample syringe to simulate injection was determined by an Instron Model No. 5564 or 5565 in compression (injection) mode using a 22 lbs. or 98 N. compression load cell at a cross head speed of 100 mm/min. The break loose force is visually determined as the highest peak of the curve or point of where the slope of the curve changes on the graph. The minimum extrusion force is determined as the lowest or smooth section of the curve after the break loose force. The extrusion force is determined by an average force of syringe plunger kinetic friction force through 20 mm movement inside syringe barrel. A typical profile of break loose and extrusion force testing is given in Figure 8. The actual test results reported in Table 1 below are an average of the test observations for thirty samples.

Example 2 (EX. 2) - Control Group II (Conventional Medium Viscosity Silicone Lubricant)

In EX. 2, components of a 5 ml. syringe were evaluated in the same manner as in EX. 1 above, however the syringe plungers were coated with a conventional polydimethylsiloxane having a viscosity of 12,500 centistokes. The conventional silicone lubricant is commercially available from Dow Chemical Company under the trade designation DC360, 12,500 cSt. The plunger material was Helvoet 5212 bromo butyl gray rubber purchased from Datwyler.

Each syringe barrel was lubricated with the same conventional polydimethylsiloxane having a viscosity of 1,000 centistokes as mentioned above.

Each syringe was assembled and filled with 5 ml of deionized water to simulate an aqueous drug solution and autoclaved at 121 °C for 30 minutes.

The test results reported in Table 1 below are an average of the test observations for thirty samples.

Example 3 (EX. 3) - Control Group III (High Viscosity Silicone Lubricant)

In EX. 3, components of a 5 ml. syringe were evaluated in the same manner as in EX. 1 above, however the syringe plungers were coated with a standalone high-viscosity hydroxyl-functional silicone having a viscosity of 30,000 centistokes.

The silicone lubricant is commercially available from the Avantor subsidiary of Nusil Technology LLC of Carpinteria, CA, under the trade designation NuSil MED-4162, 30,000 cSt. This high viscosity organopolysiloxane is mixed at 1 wt. % with a solvent of xylene and heptane at 99 wt. %. The plunger rubber material was a 5212 gray butyl rubber compound purchased from Datwyler for medical drug container applications.

Each syringe barrel was lubricated with the same conventional polydimethylsiloxane having a viscosity of 1,000 centistokes as mentioned above.

Each syringe was assembled and filled with 5 ml. of water for injection and autoclaved at 121 °C for 30 minutes. The test results reported in Table 1 below are an average of the test observations for thirty samples.

Examples 4 - 6 (EX. 4, EX. 5, EX. 6) Compositions of the Present Invention

In these examples, 5 ml. syringe components were evaluated in the same manner as in EX. 1 above, however the syringe plungers were coated with a coating of different variations of compositions of the present invention as shown in Table 1. The syringe barrels used were as previously described in EX. 1 above.

The silicone lubricant in EX. 4 is a blend of two organopolysiloxanes. The first organopolysiloxane is commercially available from Avantor under the trade designation NuSil MED10-4161, 150 cSt. The second organopolysiloxane is commercially available from Avantor under the trade designation NuSil MED-4162, 30,000 cSt. The MED10-4161, 150 cSt. organopolysiloxane is provided at 0.67 wt. % and the MED-4162 at 0.33 wt. %. The organopolysiloxanes are mixed with a solvent comprising xylene and heptane having a solvent concentration of the other 99% wt.% of the solution. The plunger rubber material was a gray Helvoet 5212 bromo butyl rubber compound purchased from Datwyler.

The silicone lubricant in EX. 5 is a blend of the same two organopolysiloxanes and solvent as EX. 4. However, the proportions of the formulation are altered such that each organopolysiloxane is provided at 1.62 wt. % and the solvent has a solvent concentration of 96.76 wt. %.

The silicone lubricant in EX. 6 is a blend of the same two organopolysiloxanes and solvent as EX. 4. However, the

proportions of the formulation are altered such that the low viscosity organopolysiloxane, with a viscosity of 150 cSt., is provided at 1.65 wt. %, the high viscosity organopolysiloxane, with a viscosity of 30,000 cSt., is provided at 3.3 wt. %, and the solvent has a solvent concentration of 95.05 wt. %.

Each syringe was assembled and filled with 5 ml of deionized water to simulate an aqueous drug solution and autoclaved at 121 °C for 30 minutes. The test results reported in Table 1 below are an average of the test results for thirty samples.

Example 7 (EX. 7) - Accelerated Aging for 50 Days at 40 °C of EX. 6 Syringe Assemblies

In this example, syringe assemblies prepared according to EX. 6 were subjected to accelerated aging conditions for 50 days at 40 °C to simulate two (2) years or 730 days at a storage temperature of 2-8 °C, which is the target shelf life and normal storage conditions for prefilled syringe assemblies. The test results reported in Table 1 below are averages of the test results for thirty samples.

Example 8 (EX. 8) - Commercial On-Market Non-Curable High-Viscosity Silicone Lubricant

In Ex. 8, components of a 5 ml. syringe were evaluated in the same manner as in EX. 1 above. However, the syringe plunger was made from Datwyler FM 457/0 bromobutyl rubber compound and was coated with a conventional high-viscosity polydimethylsiloxane having a viscosity believed to be larger than about 12,500 centistokes.

Each syringe was assembled and filled with 5 ml of deionized water to simulate aqueous drug solution and autoclaved at 121 °C for 30 minutes. The test results reported in Table 1 below are an average of the test results for thirty samples.

Example 9 (EX. 9) - Commercial On-Market Curable Silicone Lubricant

In EX. 9, components of a 5 ml. syringe were evaluated in the same manner as in EX. 1 above. However, the syringe plunger was made from West Pharmaceutical's of Kinston, NC West 4023/50 gray bromobutyl rubber compound and was coated with West Pharmaceutical curable silicone lubricant B2-44.

Each syringe was assembled and filled with 5 ml of deionized water to simulate aqueous drug solution and autoclaved at 121 °C for 30 minutes. The test results reported in Table 1 below are an average of the test observations for thirty samples.

TABLE 1

| | Sample ID | | | | | | | | |
|---|---|---|---|---|---|---|---|---|---|
| | EX. 1 | EX. 2 | EX. 3 | EX. 4 | EX. 5 | EX. 6 | EX. 7 | EX. 8 | EX. 9 |
| Formulation | | | | | | | | | |
| DC360, 1,000 cSt | 100 wt.% | | | | | | | | |
| DC360, 12,500 cSt | | 3 wt.% | | | | | | | |
| MED10-4161, 150 cSt | | | | 0.67 wt.% | 1.62 wt.% | 1.65 wt.% | 1.65 wt.% | Datwyler On-Market Non-Curable Silicone Lubricant | West On-Market B2-44 Curable Silicone Lubricant |
| MED-4162, 30,000 cSt Dispersion | | | 1 wt.% | 0.33 wt.% | 1.62 wt.% | 3.3 wt.% | 3.3 wt.% | | |
| Solvent | None | heptane | xylene and heptane | xylene and heptane | xylene and heptane | xylene and heptane | xylene and heptane | | |
| Solvent Concentration | | 97 wt.% | 99 wt.% | 99 wt.% | 96.76 wt.% | 95.05 wt.% | 95.05 wt.% | | |
| Sterilization | | | | | | | | | |
| Autoclave, °C/min | 121 °C, 30 min | | | | | | | | |

(continued)

| Function Test | | | | | | | | | |
|---|---|---|---|---|---|---|---|---|---|
| Ave. Break loose Force, N Std. Dev. in ( ) | 23.3 (5.4) | 25.1 (3.6) | 20.2 (2.7) | 11.6 (2.2) | 9.8 (0.8) | 7.33 (0.56) | 8.4 (0.6) | 38.4 (2.9) | 29.7 (1.7) |
| Stiction | Yes | Yes | Yes | No | No | No | No | Yes | Yes |
| Ave. Extrusion Force, N Std. Dev. in ( ) | 2.37 (0.6) | 4.2 (0.8) | 3.6 (1) | 7.4 (0.6) | 6.5 (0.47) | 4.11 (0.52) | 5.1 (0.52) | 1.1 (0.2) | 1.4 (0.21) |

EX. 1-3 indicate that experimentation trying conventional silicones lubricants of increasing viscosity was insufficient alone to consistently reduce break loose force and plunger stiction. This was further demonstrated by EX. 8, which was based on both a high viscosity silicone lubricant and a new plunger rubber material. EX. 9 with a commercial fully cured silicone lubricant suggests a standalone cured silicone cannot accomplish a reduced break loose and stiction, either, although it is believed a fully cured silicone molecule can significantly increase stability of silicone molecule on the plunger surface and reduce silicone's mobility and migration.

EX. 4-7 (compositions of the present invention) effectively reduced the plunger's break loose force and stiction. Without limiting the present invention, it was hypothesized that both low viscosity curable silicone I and high viscosity copolymerizable silicone II must be present together to effectively reduce the plunger's break loose force and stiction. The low viscosity curable silicone I reacts by itself and with high viscosity copolymerizable silicone II to form a 3-dimension inter-penetrated and connected network or molecular structure, which stabilizes the silicone lubricant mixture on the plunger surface to reduce migration and depletion of the lubricant. Comparing the results of EX. 7 to EX. 6 in Table 1, the fact that the break loose force and extrusion force respectively increased by relatively small amounts after a prolong period of aging, as simulated in EX. 7, is evidence of limited lubricant migration. The high viscosity copolymerizable silicone II, on one hand, can stabilize on the plunger surface due to its covalent chemical bond with low viscosity curable silicone I. On the other hand, it has a large uncured molecule section and thus a certain level of mobility even after curing reaction to allow the molecule to be able to move and flow into filling coverage of any areas on the plunger surface that are not covered by the silicone I ("dry areas"). It has been discovered that an appropriate balance between silicone stability and mobility is critical to achieve maximum reduction of the break loose force and stiction, which can be achieved by adjusting viscosity between the two silicone molecules and their ratio in the blend. The silicone I must also simultaneously be highly curable via a low viscosity and maximize the concentration of the curing function group for curing both with itself and silicone II.

Thus, it has been discovered that when reactive functionality is equal to or less than two, the silicone molecule is only able to grow linearly in two-dimension direction, and one or a mixture of two such "curable" silicones fail to form a 3-dimension network. For a non-curable silicone molecule, it will not form a semi interpenetrating network with a 3-dimension crosslinked silicone until its molecular weight is large enough to be able to penetrate through and entangle the crosslinked 3D network. However, 3-dimension fully or semi interpenetrating network is preferred to reduce silicone mobility and help stabilize silicone coating on the substrate surface for an extended time.

FIG. 8 illustrates plunger movement in millimeters versus force in Newtons applied to the plunger for a representative case of one commercially available syringe with a conventional lubricant. FIG. 8A illustrates the improved results discovered using the lubricant of the present invention and relates to the embodiment of EX. 6 from Table 1. The average break loose force is reduced to 7.33 Newtons (N) and the average extrusion force has been reduced to 4.11 N.

FIG. 9 is a bar chart showing the average (mean) break loose force comparison testing between the present invention (EX. 6) and on-market syringe plunger/lubricant combinations (EX. 8 and 9) from Table 1. The lubricant of the invention provided break loose force in single digits at 7.33 N., while the on-market combinations exhibited higher break loose forces, far into double digits, at 38.4 N. and 29.7 N. respectively. FIG. 10 is a bar chart showing the calculated break loose force minus extrusion force values for the invention (EX. 6) and the same two on-market plunger/syringe combinations from EX. 8 and 9 in Table 1. The difference in the EX. 6 case of the invention is (7.33-4.11) = 3.22 N. compared to (38.4-1.1) = 37.3 N. for EX. 8 and (29.7-1.4) = 28.3 N. for EX. 9. The lubricant of the present invention provides a relatively smooth force feedback or "feel" to the user during operation, which compares favorably to the potentially somewhat jerky motion or feel of many on-market plunger/lubricant combinations. The smoother feel allows the caregiver or other user to more easily dispense doses and even dispense doses in partial doses or small increments if needed. Another factor that contributes to the feel for the caregiver or user is the ratio of the break loose force to the extrusion force. FIG. 11 is a bar chart showing the calculated ratio of break loose force to extrusion force values for the invention as embodied in EX. 6 versus the on-market plunger/lubricant combinations of EX. 8 and 9 from Table 1. The lubricant of the present invention yields a ratio of 7.33/4.11 = 1.78, while EX. 8 and 9 have ratios of 38.4/1.1 = 34.91 and 29.7/1.4 = 21.21 respectively. Thus, the lubricant and the lubricant/plunger combination of the invention provides a ratio of break loose force

to extrusion force of less than 3, or between approximately 1 and about 3, and more particularly between about 1.5 (EX. 5) and about 2 (EX. 6). Surprisingly, even when subjected to accelerated aging conditions, such as in EX. 7, the ratio stays in this range at 1.65 and the break loose force did not increase by more than 20 percent. Maintaining this low ratio provides a more predictable and consistent feedback to the caregiver or user, which allows them to dispense doses of medicaments more accurately.

Example 10 (EX. 10) Compositions of the Present Invention

The silicone lubricant in EX. 10 is a blend of the same two organopolysiloxanes and solvent as EX. 6 and EX.7. However, the proportions of the formulation are altered such that the low viscosity organopolysiloxane, with a viscosity of 150 cSt., is provided at 5 wt. %, the high viscosity organopolysiloxane, with a viscosity of 30,000 cSt., is provided at 10 wt. %, and the solvent has a solvent concentration of 85 wt. %.

Each syringe was assembled and filled with 5 ml of deionized water to simulate aqueous drug solution and autoclaved at 121 °C for 30 minutes. The test results reported in Table 2 below are an average of the test observations for thirty samples.

Examples 11 - 13 (EX. 11, EX. 12, EX. 13) Compositions of the Present Invention

The silicone lubricant in EX. 11 is a blend of two organopolysiloxanes. The first organopolysiloxane is commercially available from Avantor under the trade designation NuSil MED10-4161, 150 cSt. The second hydroxyl-functional organopolysiloxane is commercially available from Gelest under the trade designation Gelest DMS-S35 (5,000 cSt). The MED10-4161, 150 cSt. organopolysiloxane is provided at 5 wt. % and the DMS-S35 at 3.3 wt. %. The organopolysiloxanes are mixed with a solvent of isopropyl alcohol having a solvent concentration of 91.7 wt.% of the solution.

The silicone lubricant in EX. 12 is a blend of two organopolysiloxanes. The first organopolysiloxane is commercially available from Avantor under the trade designation NuSil MED10-4161, 150 cSt. The second hydroxyl-functional organopolysiloxane is commercially available from Gelest under the trade designation Gelest DMS-S42 (18,000 cSt). The MED10-4161, 150 cSt. organopolysiloxane is provided at 5 wt. % and the DMS-S42 at 3.3 wt. %. The organopolysiloxanes are mixed with a solvent of isopropyl alcohol having a solvent concentration of 91.7 wt.% of the solution.

The silicone lubricant in EX. 13 is a blend of two organopolysiloxanes. The first organopolysiloxane is commercially available from Avantor under the trade designation NuSil MED10-4161, 150 cSt. The second hydroxyl-functional organopolysiloxane is commercially available from Gelest under the trade designation Gelest DMS-S45 (50,000 cSt). The MED10-4161, 150 cSt. organopolysiloxane is provided at 5 wt. % and the DMS-S45 at 3.3 wt. %. The organopolysiloxanes are mixed with a solvent of isopropyl alcohol having a solvent concentration of 91.7 wt.% of the solution.

Examples 14 - 16 (EX. 14, EX. 15, EX. 16) Compositions of the Present Invention

In these examples, syringes were evaluated in the same manner as in EX. 10 above, however the syringe plungers were coated with a with a lubricant formulation, composition or blend including a third conventional polydimethylsiloxane having a viscosity of 1,000 centistokes. The conventional silicone lubricant is commercially available from Dow Chemical Company under the trade designation DC360, 1,000 cSt.

The silicone lubricant in EX. 14 is a blend of three organopolysiloxanes. The first organopolysiloxane is commercially available from Avantor under the trade designation NuSil MED10-4161, 150 cSt. The second organopolysiloxane is commercially available from Avantor under the trade designation NuSil MED-4162, 30,000 cSt. The third organopolysiloxane is commercially available from Dow Chemical Company under the trade designation DC360, 1,000 cSt. The MED10-4161, 150 cSt. organopolysiloxane is provided at 1.5 wt. %, the MED-4162 at 3.0 wt. % and the DC360 1000 cSt at 0.5 wt. %. The organopolysiloxanes are mixed with a solvent of heptane having a solvent concentration of the other 95 wt.% of the solution.

The silicone lubricant in EX. 15 is a blend of the same three organopolysiloxanes and solvent as EX. 14. However, the proportions of the formulation are altered such that the low viscosity organopolysiloxane, with a viscosity of 150 cSt., is provided at 2.5 wt. %, the medium viscosity organopolysiloxane, with a viscosity of 1,000 cSt., is provided at 0.5 wt. %, the high viscosity organopolysiloxane, with a viscosity of 30,000 cSt., is provided at 5 wt. %, and the solvent has a solvent concentration of 89.5 wt. %.

The silicone lubricant in EX. 16 is a blend of the same three organopolysiloxanes and solvent as EX. 14. However, the proportions of the formulation are altered such that the low viscosity organopolysiloxane, with a viscosity of 150 cSt., is provided at 2.5 wt. %, the medium viscosity organopolysiloxane, with a viscosity of 1,000 cSt., is provided at 0.75 wt. %, the

high viscosity organopolysiloxane, with a viscosity of 30,000 cSt., is provided at 5 wt. %, and the solvent has a solvent concentration of 91.75 wt. %.

TABLE 2

| | Sample ID | | | | | | |
|---|---|---|---|---|---|---|---|
| | EX. 10 | EX. 11 | EX. 12 | EX. 13 | EX. 14 | EX. 15 | EX. 16 |
| DC360, 1,000 cSt | | | | | 0.5 wt% | 3 wt% | 0.75 wt% |
| Gelest DMS-S35 (5,000 cSt) | | 3.3 wt% | | | | | |
| Gelest DMS-S42 (18,000 cSt) | | | 3.3 wt% | | | | |
| Gelest DMS-S45 (50,000 cSt) | | | | 3.3 wt% | | | |
| MED10-4161 | 5 wt% | 5 wt% | 5 wt% | 5 wt% | 1.5 wt% | 2.5 wt% | 2.5 wt% |
| MED-4162, 30,000 cSt Dispersion | 10 wt% | | | | 3 wt% | 5 wt% | 5 wt% |
| Solvent | Heptane | Isopropyl Alcohol | Heptane | Heptane | Heptane | Heptane | Heptane |
| Solvent Concentration | 85 wt% | 91.7 wt% | 91.7 wt% | 91.7 wt% | 95 wt% | 89.5 wt% | 91.75 wt% |
| Sterilization | | | | | | | |
| Autoclave, °C/min | 121 °C, 30 min | | | | | | |
| Function Test | | | | | | | |
| Break loose Force, N; Std. Dev. in ( ) | 6.59 (0.5) | 8.43 (0.46) | 7.9 (0.35) | 7.23 (0.43) | 8.45 (1.0) | 6.69 (0.46) | 7.2 (0.45) |
| Sticktion | No | No | No | No | No | No | No |
| Extrusion Force, N; Std. Dev. in ( ) | 3.62 (0.43) | 4.62 (0.39) | 4.32 (0.4) | 3.87 (0.6) | 2.9 (0.51) | 2.92 (0.52) | 3.9 (0.9) |

EX. 10-16 indicate that combining two or even three organopolysiloxanes of curable and non-curable, but copolymerizable, varieties to form a lubricant formulation reduced break loose force, the difference between extrusion force and break loose force, the ratio of break loose force to extrusion force, and little to no plunger stiction. As evidenced by EX. 11, the viscosity of the silicone II molecule can be equal to or greater than about 5,000 centistokes and the formulation still yields low break loose force (8.43 N) and low extrusion force (4.62 N). Interestingly as shown in EX. 14-16, adding a third, more traditional or conventional silicone DC360, 1,000 cSt, which is non-curable and non-copolymerizable, does not adversely impact the development of the interpenetrating network of this invention and the results achieved. Advantageously, the silicone components of the invention show good compatibility with the traditional silicone DC360, 1,000 cSt.

FIG. 12 is a bar chart showing the average (mean) break loose force comparison between these embodiments of the present invention (EX. 10-13) from Table 2 and the existing and on-market syringe plunger/lubricant combinations (EX. 1, 8 and 9) from Table 1. The lubricant of these embodiments of the invention provided break loose force in single digits from 6.59 N to 8.43 N, while the existing and on-market combinations exhibited higher break loose forces, far into double digits, at 23.3 N, 38.4 N. and 29.7 N. respectively. FIG. 13 is a bar chart showing the average (mean) break loose force comparison testing between other embodiments of the present invention (EX. 14-16) from Table 2 and the existing and on-market syringe plunger/lubricant combinations (EX. 1, 8 and 9) from Table 1. The lubricant of these embodiments of the invention still provided break loose force in single digits from 6.69 N to 8.45 N, while the existing and on-market combinations exhibited higher break loose forces, far into double digits, at 23.3 N, 38.4 N. and 29.7 N. respectively. As seen in EX. 14-16 of Table 2, including some conventional or traditional silicone DC360, 1,000 cSt, which is non-curable and non-copolymerizable, does not significantly increase the observed break loose force. The lubricant of the present invention provides a relatively smooth force feedback or "feel" to the user during operation, which compares favorably to the potentially somewhat jerky motion or feel of many on-market plunger/lubricant combinations. The smoother feel allows the caregiver or other user to more easily dispense doses and even dispense doses in partial doses or small increments if

needed.

One factor that contributes to the feel for the caregiver or user is the difference between the break loose force and the extrusion force. In EX. 10-16 the differential values in Newtons are 6.59-3.62 = 2.97; 8.43-4.62 = 3.81; 7.9-4.32 = 3.58; 7.23-3.87 = 3.36; 8.45-2.9 = 5.55; 6.69-2.92 = 3.77; 7.2-3.9 = 3.3, respectively. Thus, the differential value is generally less than or equal to 6 N. Another factor that contributes to the feel for the caregiver or user is the ratio of the break loose force to the extrusion force. The ratios of the break loose force to extrusion force remains low in the embodiments of EX. 10-16 as compared to the existing and on-market plunger/lubricant combinations of EX. 1, 8 and 9 from Table 1. In EX. 10-16, the lubricant of the present invention yields a ratio of 6.59/3.62 = 1.82; 8.43/4.62 = 1.82; 7.9/4.32 = 1.83; 7.23/3.87 = 1.87; 8.45/2.9 = 2.9; 6.69/2.92 = 2.29; 7.2/3.9 = 1.85, respectively, while EX. 1, 8 and 9 have ratios of 23.3/2.37 = 9.83; 38.4/1.1 = 34.91; and 29.7/1.4 = 21.21 respectively. Thus, the lubricant and the lubricant/plunger combination of the invention provides a ratio of break loose force to extrusion force of less than 3, or between approximately 1 and about 3. Maintaining this low ratio provides a more predictable and consistent feedback to the caregiver or user, which allows them to dispense doses of medicaments more accurately.

Based upon the foregoing it should be apparent that the present invention at least satisfies its stated objectives. A low cost, easy to manufacture, blend, apply and cure, silicone lubricant formulation is disclosed, which yields a low break loose force that is less than two times the extrusion force and thereby reduces stiction between a first surface and a second surface when applied to one of the surfaces. The average break loose force with the inventive formulation is low and tightly controllable in manufacturing, as indicated by the low standard deviation observed in the tests. The extrusion force is moderate, which is desirable to minimize the chances of unintended or premature plunger movement. In other words, users also desire some force feedback as they press on the plunger or plunger rod but do not want it to move prematurely.

The new formulation of lubricant coating described herein with respect to coating the plunger component can be used to coat the syringe barrel instead of or in addition to the plunger and similar improved break loose and stiction reduction is expected.

## Claims

1. A syringe comprising:

    a tubular syringe barrel having an interior surface;
    a plunger slidingly mountable within the syringe barrel and having an outwardly directed surface for slidably engaging the interior surface of the syringe barrel;
    the interior surface of the syringe barrel and the plunger defining a container chamber configured to contain a medicament;
    a lubricious coating applied to at least one of the syringe barrel interior surface and the outwardly directed surface of the plunger; and
    wherein the lubricious coating is a mixture of at least two silicones comprising a first silicone being a hydrolyzable siloxane capable of a crosslinking reaction upon exposure to moisture at ambient temperature and a second silicone being an organopolysiloxane that is copolymerizable with the first silicone.

2. The syringe of claim 1, wherein when a force is applied to the plunger by a user to slidably move the plunger within the syringe barrel, a break loose force to be overcome to start movement of the plunger within the syringe barrel is less than or equal to three times an extrusion force to be overcome when the plunger is already in motion.

3. The syringe of claim 2, wherein the extrusion force is less than or equal to 6 N.

4. The syringe of claims 2 or 3, wherein the break loose force is less than or equal to 15 N.

5. The syringe of any of claims 2-4, wherein a secondary break loose force, which is encountered when a second force is applied to the plunger after the plunger has been parked or stationary following withdrawal of a first applied force, is less than or equal to 10 N.

6. The syringe of claim 5, wherein the secondary break loose force is no more than 6 N less than the break loose force.

7. The syringe of any of claims 1-6, wherein the outwardly directed surface of the plunger comprises an elastomeric material that is coated with the lubricious coating.

8. The syringe of any of claims 1-7, wherein the coating is applied to a thickness from about 0.1 μm to 40 μm.

9. The syringe of any of claims 1-8 containing a pharmaceutically active ingredient, wherein the pharmaceutically active ingredient is selected from a group consisting of rocuronium bromide, ephedrine, and succinylcholine chloride.

## Patentansprüche

1. Spritze, umfassend: einen röhrenförmigen Spritzenzylinder mit einer Innenfläche;

   einen Kolben, der gleitend innerhalb des Spritzenzylinders montierbar ist und eine nach außen gerichtete Fläche zum gleitenden Eingreifen in die Innenfläche des Spritzenzylinders aufweist;
   wobei die Innenfläche des Spritzenzylinders und der Kolben eine Behälterkammer definieren, die konfiguriert ist, um ein Medikament zu enthalten;
   eine gleitfähige Beschichtung, die auf mindestens eine von der Innenfläche des Spritzenzylinders und der nach außen gerichteten Fläche des Kolbens aufgebracht ist; und
   wobei die gleitfähige Beschichtung eine Mischung aus mindestens zwei Silikonen ist, umfassend ein erstes Silikon, das ein hydrolysierbares Siloxan ist, das bei Exposition gegenüber Feuchtigkeit bei Umgebungstemperatur zu einer Vernetzungsreaktion fähig ist, und ein zweites Silikon, das ein Organopolysiloxan ist, das mit dem ersten Silikon copolymerisierbar ist.

2. Spritze nach Anspruch 1, wobei, wenn von einem Benutzer eine Kraft auf den Kolben ausgeübt wird, um den Kolben gleitend innerhalb des Spritzenzylinders zu bewegen, eine Losbrechkraft, die zu überwinden ist, um die Bewegung des Kolbens innerhalb des Spritzenzylinders zu beginnen, kleiner als oder gleich dem Dreifachen einer Extrusionskraft ist, die zu überwinden ist, wenn der Kolben bereits in Bewegung ist.

3. Spritze nach Anspruch 2, wobei die Extrusionskraft kleiner als oder gleich 6 N ist.

4. Spritze nach Anspruch 2 oder 3, wobei die Losbrechkraft kleiner als oder gleich 15 N ist.

5. Spritze nach einem der Ansprüche 2-4, wobei eine sekundäre Losbrechkraft, die auftritt, wenn eine zweite Kraft auf den Kolben ausgeübt wird, nachdem der Kolben nach dem Zurückziehen einer ersten ausgeübten Kraft geparkt oder stationär wurde, kleiner als oder gleich 10 N ist.

6. Spritze nach Anspruch 5, wobei die sekundäre Losbrechkraft nicht mehr als 6 N kleiner als die Losbrechkraft ist.

7. Spritze nach einem der Ansprüche 1-6, wobei die nach außen gerichtete Fläche des Kolbens ein elastomeres Material umfasst, das mit der gleitfähigen Beschichtung beschichtet ist.

8. Spritze nach einem der Ansprüche 1-7, wobei die Beschichtung in einer Dicke von etwa 0,1 $\mu$m bis 40 $\mu$m aufgebracht ist.

9. Spritze nach einem der Ansprüche 1-8, enthaltend einen pharmazeutischen Wirkstoff, wobei der pharmazeutische Wirkstoff ausgewählt ist aus einer Gruppe bestehend aus Rocuroniumbromid, Ephedrin und Succinylcholinchlorid.

## Revendications

1. Seringue, comprenant :

   un corps de seringue tubulaire ayant une surface intérieure ;
   un plongeur pouvant être monté de manière coulissante au sein du corps de seringue et ayant une surface dirigée vers l'extérieur pour s'engager de manière coulissante avec la surface intérieure du corps de seringue ;
   la surface intérieure du corps de seringue et le plongeur définissant une chambre de contenant configurée pour contenir un médicament ;
   un revêtement lubrifiant appliqué sur au moins l'une de la surface intérieure du corps de seringue et de la surface dirigée vers l'extérieur du plongeur ; et
   dans laquelle le revêtement lubrifiant est un mélange d'au moins deux silicones comprenant un premier silicone étant un siloxane hydrolysable capable d'une réaction de réticulation lors d'une exposition à de l'humidité à température ambiante, et un deuxième silicone étant un organopolysiloxane qui est copolymérisable avec le

premier silicone.

2. Seringue selon la revendication 1, dans laquelle, lorsqu'une force est appliquée au plongeur par un utilisateur pour mettre en mouvement le plongeur de manière coulissante au sein du corps de seringue, une force de décollage à surmonter pour démarrer le mouvement du plongeur au sein du corps de seringue est inférieure ou égale à trois fois une force d'extrusion à surmonter lorsque le plongeur est déjà en mouvement.

3. Seringue selon la revendication 2, dans laquelle la force d'extrusion est inférieure ou égale à 6 N.

4. Seringue selon les revendications 2 ou 3, dans laquelle la force de décollage est inférieure ou égale à 15 N.

5. Seringue selon l'une des revendications 2 à 4, dans laquelle une force de décollage secondaire, qui est rencontrée lorsqu'une seconde force est appliquée au plongeur après que le plongeur a été immobilisé ou arrêté suite à un retrait d'une première force appliquée, est inférieure ou égale à 10 N.

6. Seringue selon la revendication 5, dans laquelle la force de décollage secondaire n'est pas inférieure de plus de 6 N à la force de décollage.

7. Seringue selon l'une des revendications 1 à 6, dans laquelle la surface dirigée vers l'extérieur du plongeur comprend un matériau élastomérique qui est revêtu du revêtement lubrifiant.

8. Seringue selon l'une des revendications 1 à 7, dans laquelle le revêtement est appliqué sur une épaisseur d'environ 0,1 $\mu$m à 40 $\mu$m.

9. Seringue selon l'une des revendications 1 à 8 contenant un ingrédient pharmaceutiquement actif, dans laquelle l'ingrédient pharmaceutiquement actif est choisi dans un groupe consistant en du bromure de rocuronium, de l'éphédrine et du chlorure de succinylcholine.

FIG. 1

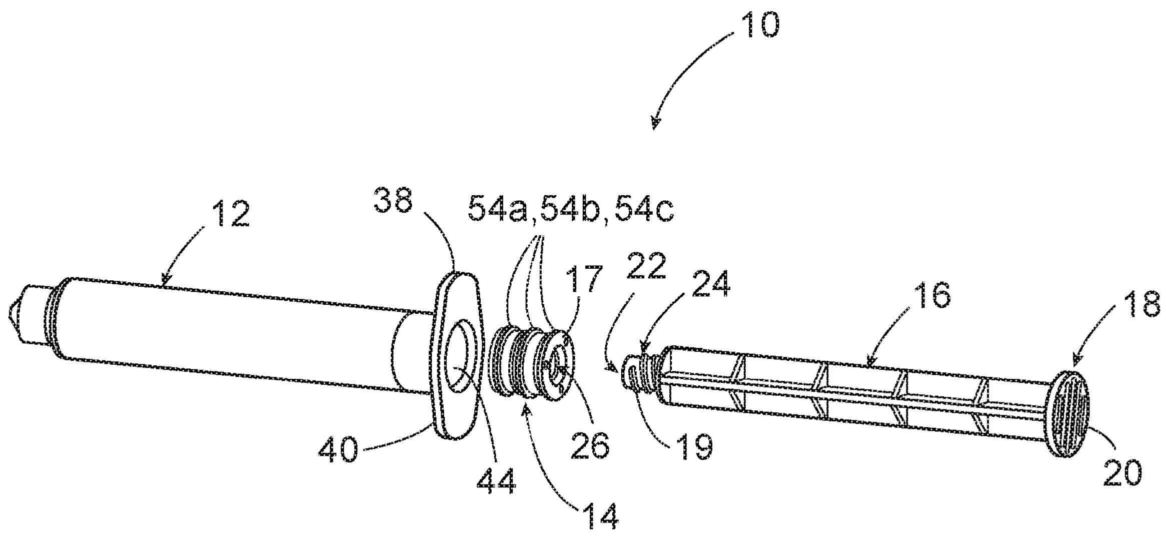

# FIG. 2

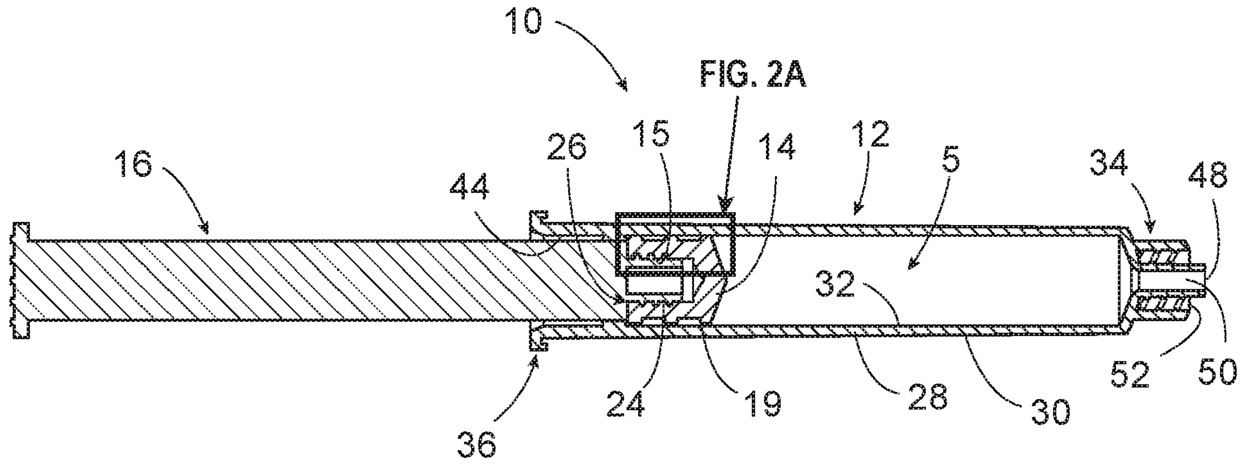
10
FIG. 2A
16
44
26
15
14
12
5
34
48
32
52
50
36
24
19
28
30

# FIG. 2A

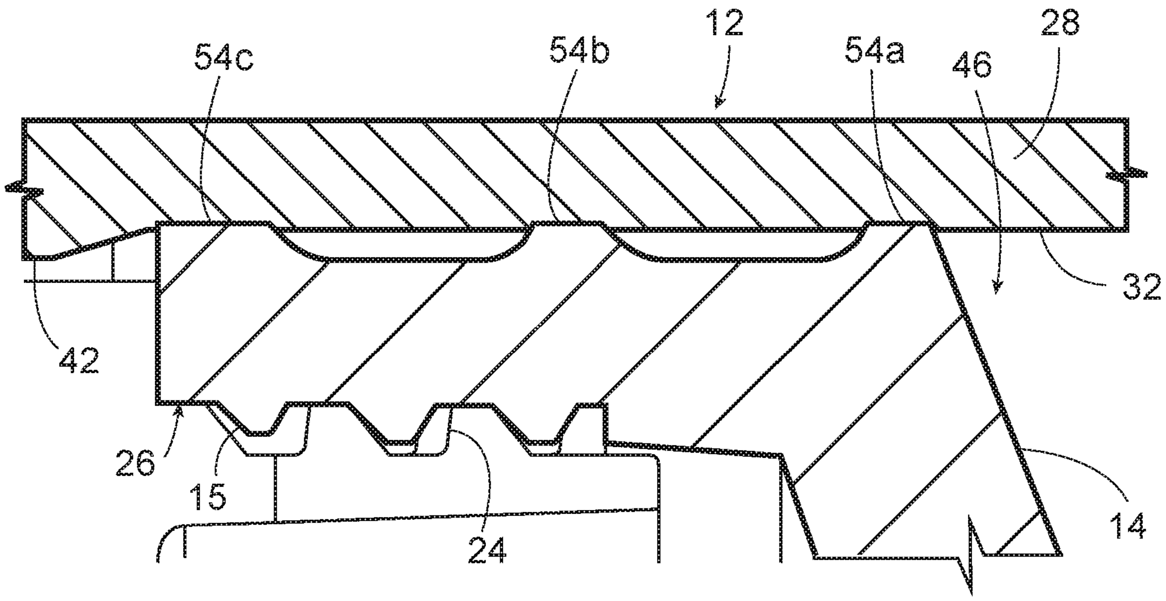
12
28
54c
54b
54a
46
32
42
26
15
24
14

# FIG. 3

Molecule 1

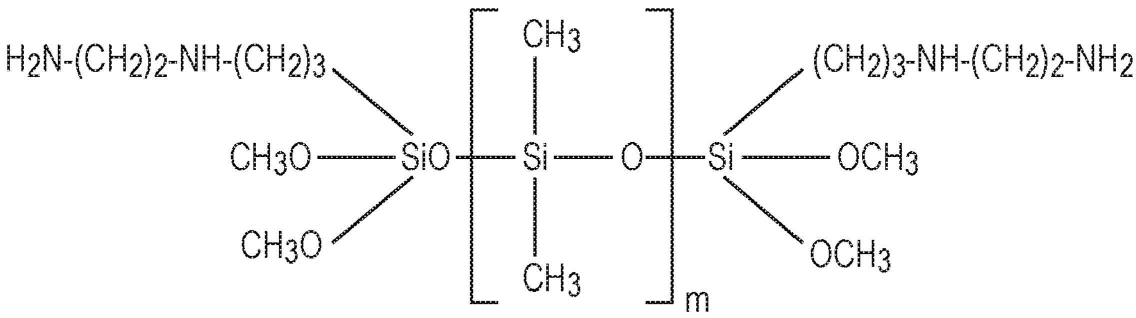

Molecule 2

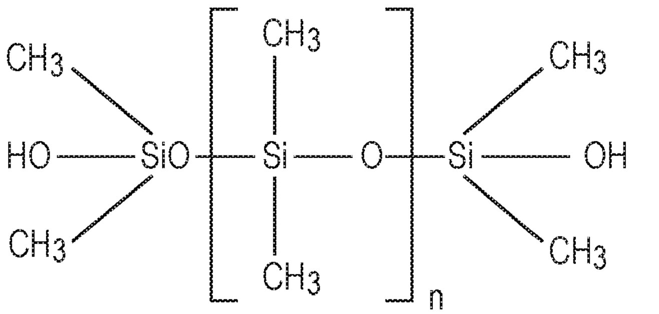

FIG. 4

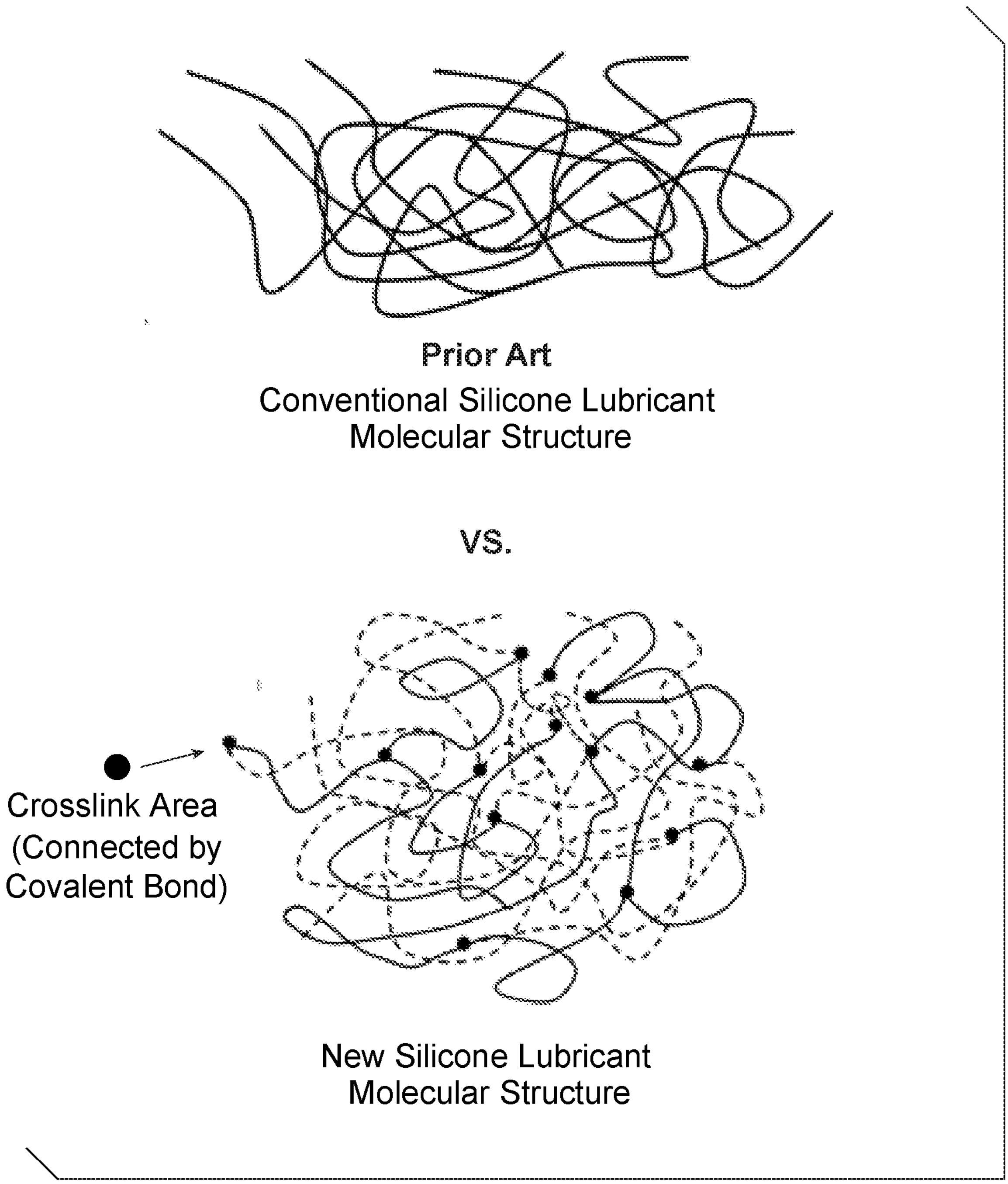
Prior Art
Conventional Silicone Lubricant
Molecular Structure
VS.
Crosslink Area
(Connected by
Covalent Bond)
New Silicone Lubricant
Molecular Structure

FIG. 5

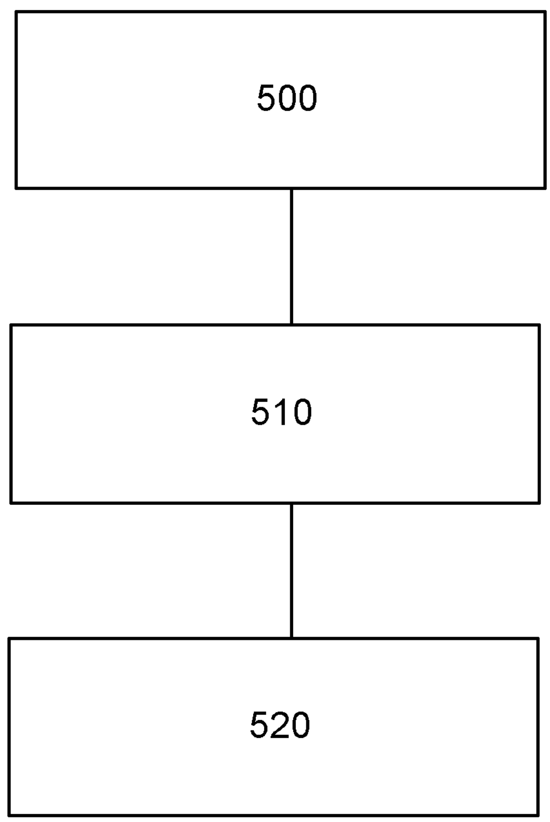

FIG. 6

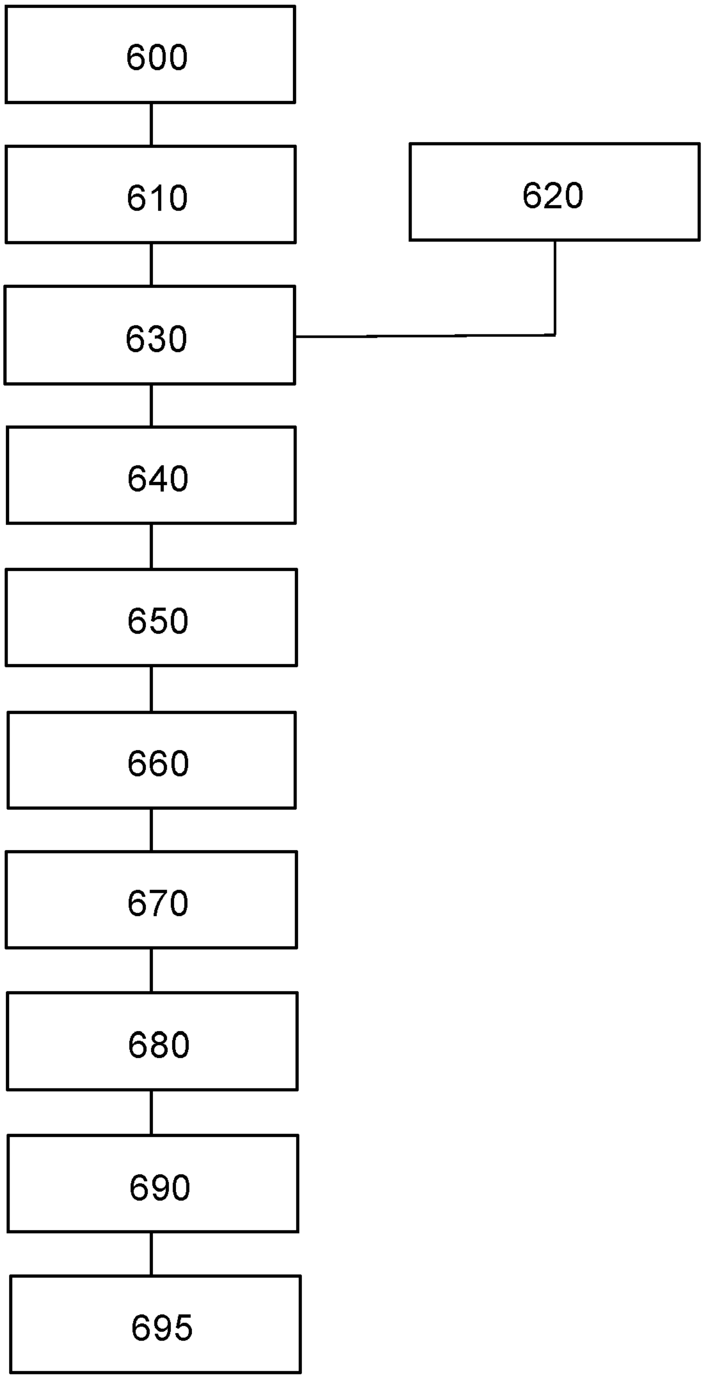

FIG. 7

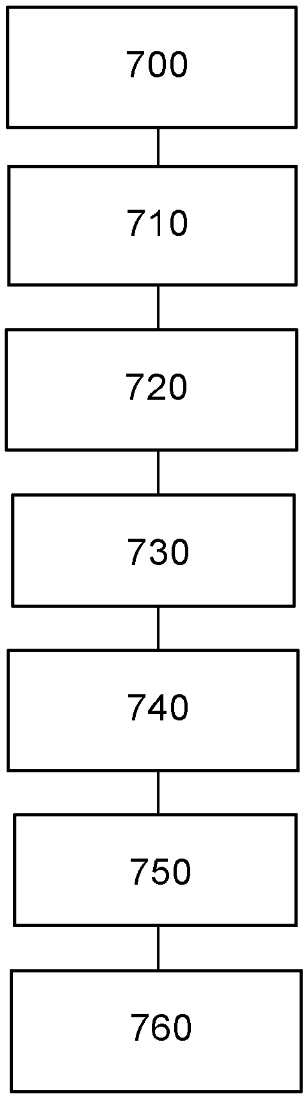

FIG. 8

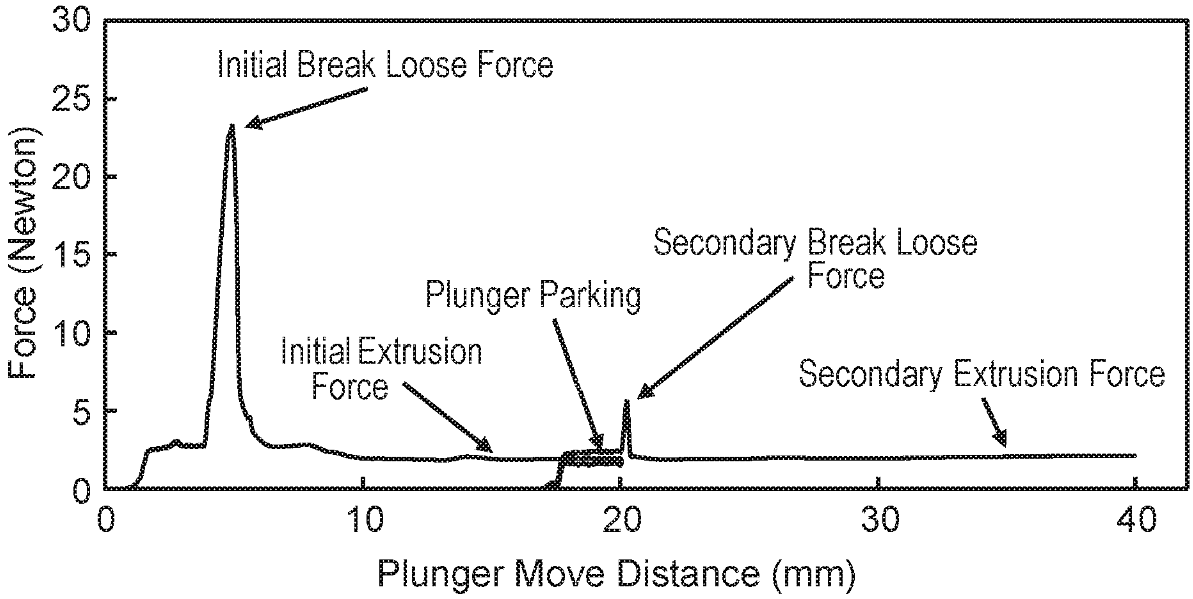
Initial Break Loose Force
Initial Extrusion Force
Plunger Parking
Secondary Break Loose Force
Secondary Extrusion Force
Force (Newton)
Plunger Move Distance (mm)
0
5
10
15
20
25
30
0
10
20
30
40

FIG. 8A

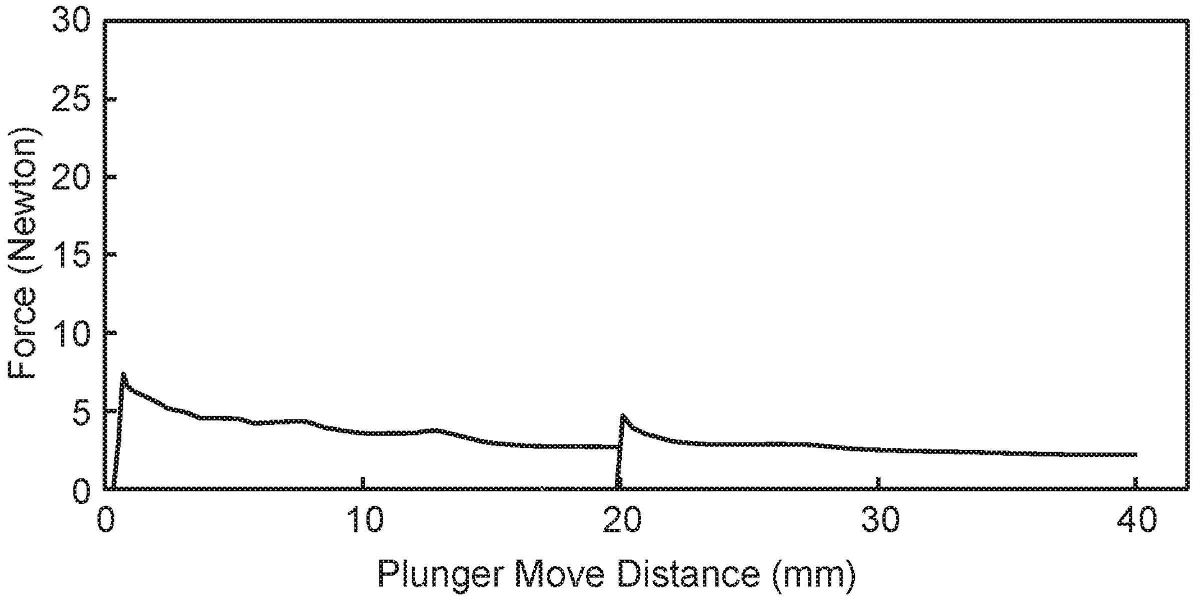
30
25
20
15
10
5
0
Force (Newton)
0
10
20
30
40
Plunger Move Distance (mm)

FIG. 9

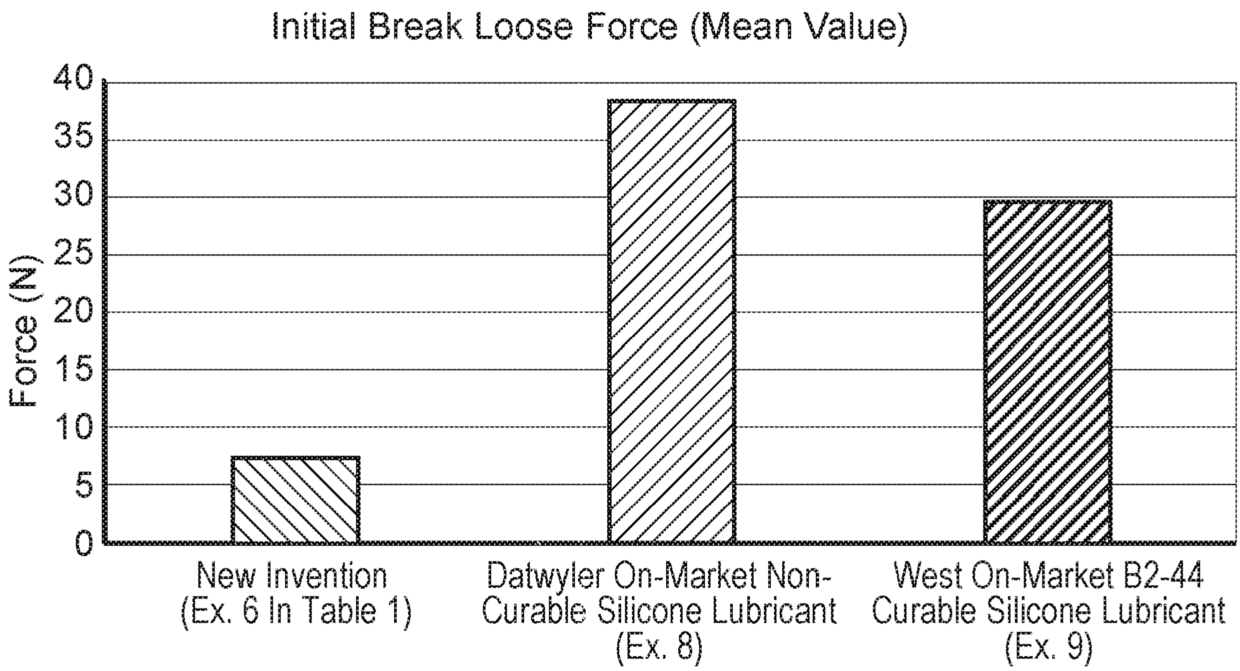
Initial Break Loose Force (Mean Value)
Force (N)
40
35
30
25
20
15
10
5
0
New Invention (Ex. 6 In Table 1)
Datwyler On-Market Non-Curable Silicone Lubricant (Ex. 8)
West On-Market B2-44 Curable Silicone Lubricant (Ex. 9)

# FIG. 10

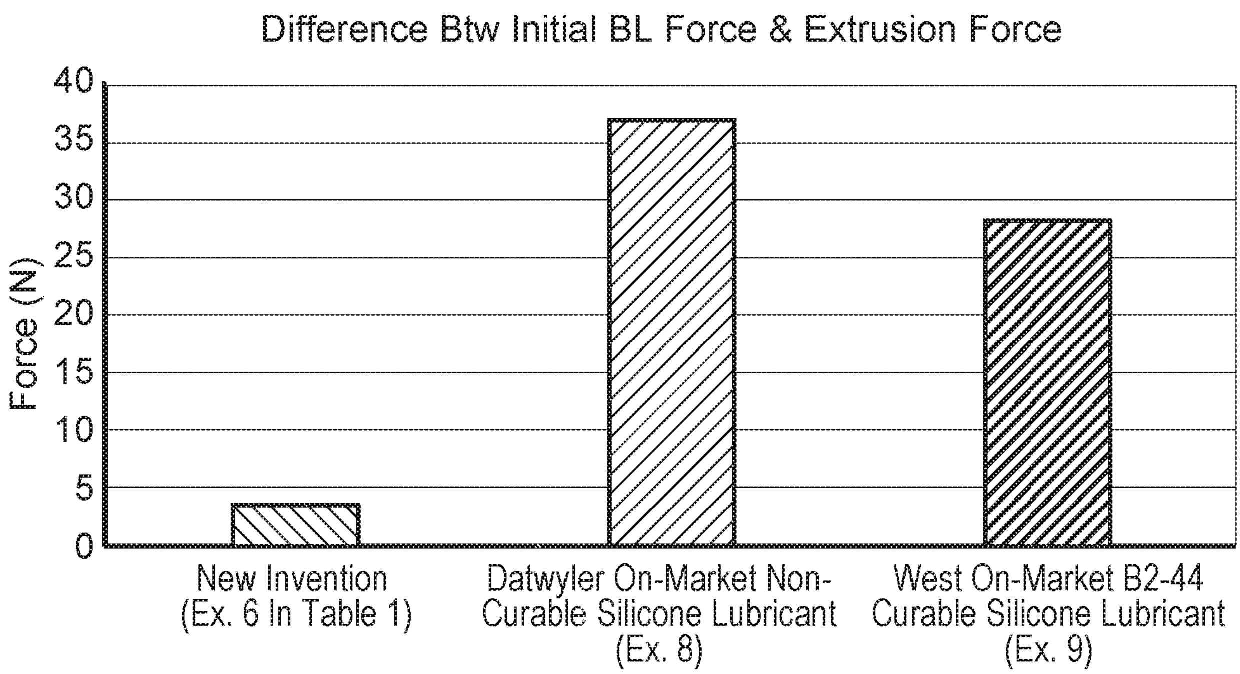
Difference Btw Initial BL Force & Extrusion Force
Force (N)
40
35
30
25
20
15
10
5
0
New Invention (Ex. 6 In Table 1)
Datwyler On-Market Non-Curable Silicone Lubricant (Ex. 8)
West On-Market B2-44 Curable Silicone Lubricant (Ex. 9)

FIG. 11

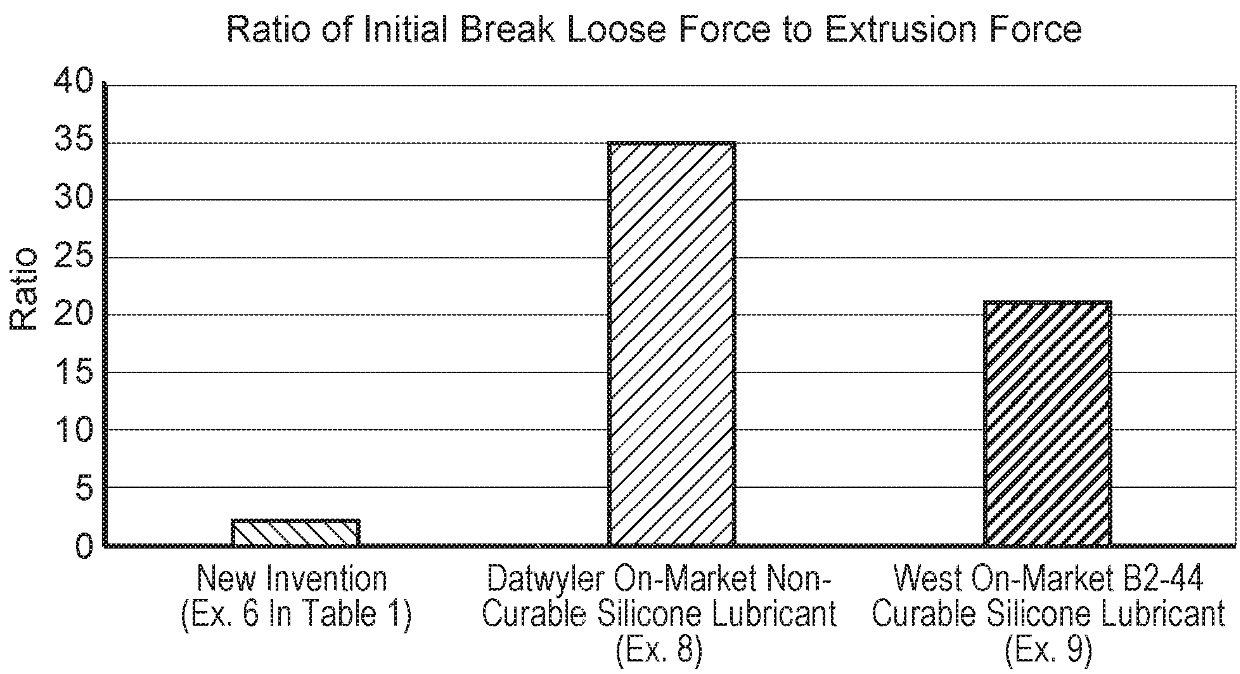
Ratio of Initial Break Loose Force to Extrusion Force
40
35
30
25
20
15
10
5
0
Ratio
New Invention
(Ex. 6 In Table 1)
Datwyler On-Market Non-
Curable Silicone Lubricant
(Ex. 8)
West On-Market B2-44
Curable Silicone Lubricant
(Ex. 9)

FIG. 12

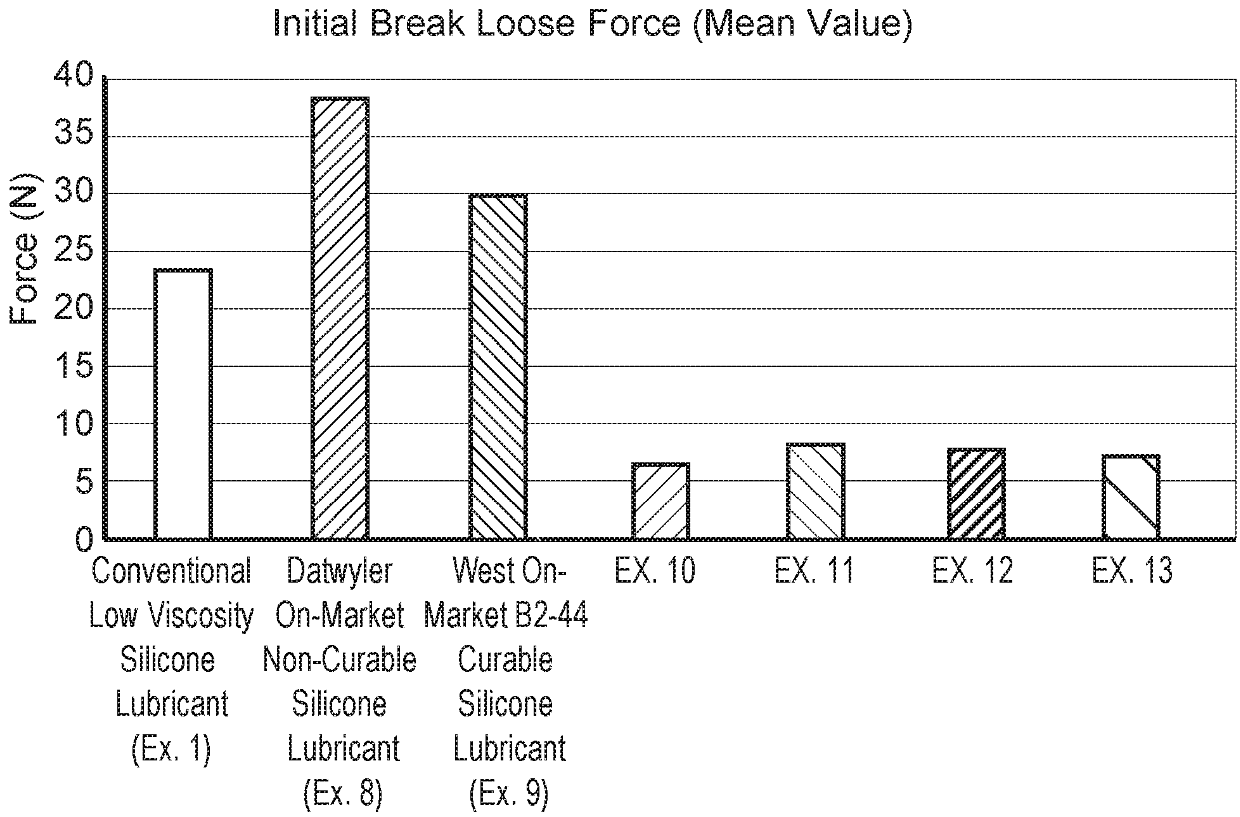
Initial Break Loose Force (Mean Value)
Force (N)
40
35
30
25
20
15
10
5
0
Conventional Low Viscosity Silicone Lubricant (Ex. 1)
Datwyler On-Market Non-Curable Silicone Lubricant (Ex. 8)
West On-Market B2-44 Curable Silicone Lubricant (Ex. 9)
EX. 10
EX. 11
EX. 12
EX. 13

# FIG. 13

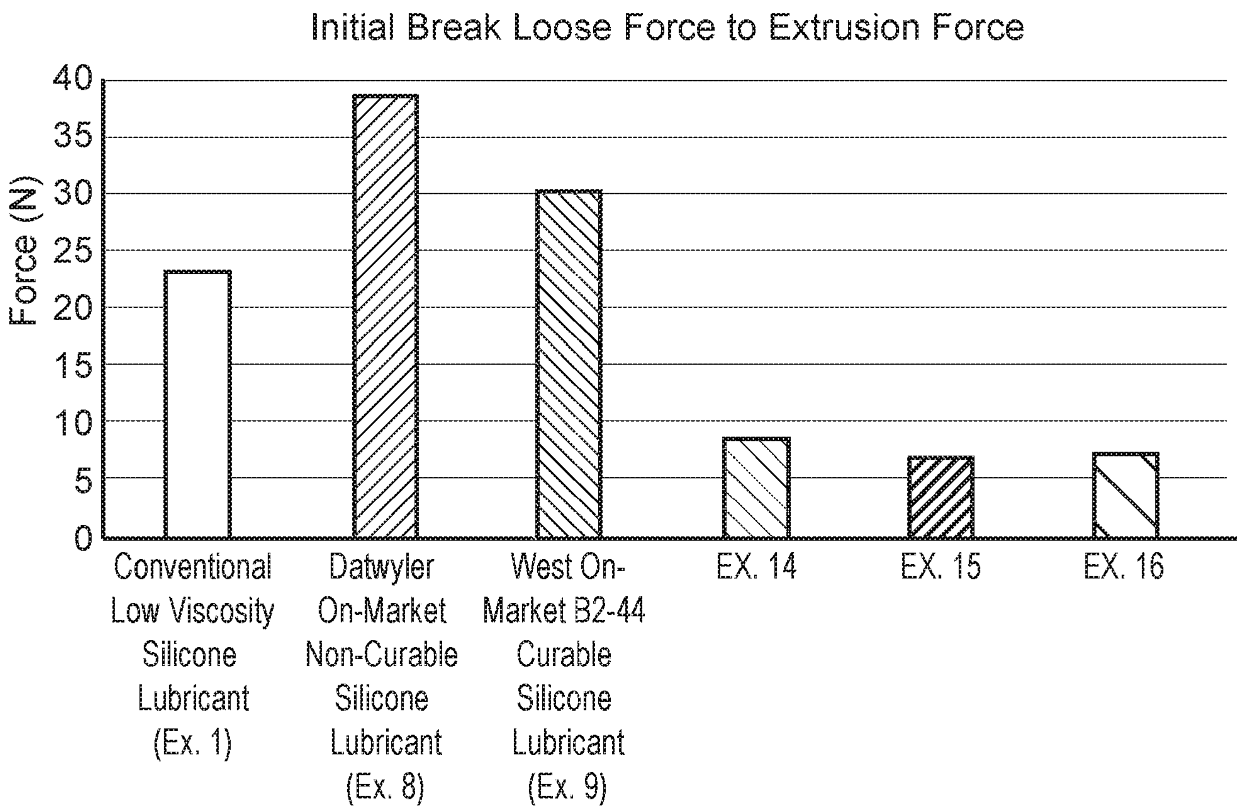
Initial Break Loose Force to Extrusion Force
Force (N)
40
35
30
25
20
15
10
5
0
Conventional Low Viscosity Silicone Lubricant (Ex. 1)
Datwyler On-Market Non-Curable Silicone Lubricant (Ex. 8)
West On-Market B2-44 Curable Silicone Lubricant (Ex. 9)
EX. 14
EX. 15
EX. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7553529 B
- US 7431989 B
- US 8124207 B
- US 9133412 B
- US 9315757 B
- US 7332227 B
- US 8075995 B
- US 9234118 B
- US 8603638 B
- US 8816022 B
- EP 2061529 A
- US 4720521 A
- US 9434857 B
- US 5908686 A
- US 6890345 B
- US 4959402 A
- US 4994552 A